(19) Europäisches Patentamt
European Patent Office
Office européen des brevets

(11) Publication number: **0 273 889**
**A2**

(12) **EUROPEAN PATENT APPLICATION**

(21) Application number: 87850379.6

(22) Date of filing: 07.12.87

(51) Int. Cl.⁴: **C 12 N 15/00**
C 12 P 21/02, C 12 N 9/02,
C 12 N 5/00

(30) Priority: 30.12.86 SE 8605621

(43) Date of publication of application:
06.07.88 Bulletin 88/27

(84) Designated Contracting States:
AT BE CH DE FR GB IT LI NL SE

(71) Applicant: **Genelux AB**
**P.O. Box 2142**
**S-183 02 Täby (SE)**

(72) Inventor: **Olsson, Olof**
**University of Umea Unit for Applied Cell**
**and Molecular Biology S-901 87 Umea (SE)**

(74) Representative: **Henningsson, Gunnar et al**
**Bergling & Sundbergh AB Box 7645**
**S-103 94 Stockholm (SE)**

The microorganism(s) has (have) been deposited with Deutsche Sammlung von Mikroorganismen under number(s) 3920.

(54) **Heteromultimeric proteins and their manufacture.**

(57) A method of in vivo production of a heteromultimeric protein, the subunits of which together but not separately are capable of generating a specific bioactivity comprising the steps:

a) isolating at least two procaryotic genes coding for separate polypeptides;

b) eliminating all ATG:s on the 5' end of each gene except the one coding for the starting methionine;

c) cloning the genes resulting from step b) into at least one eucaryotic expression vector;

d) introducing the expression vector(s) from step c) into a eucaryotic cell; and

e) allowing said cell to express separately but simultaneously and coordinately the protein subunits coded for by the said genes resulting in generation of said specific bioactivity;

a eucaryotic plasmid vector having introduced therein procaryotic DNA-constructs coding for the separate expression of the subunits of a heteromultimeric protein capable of generating a specific bioactivity in vivo; and

the corresponding DNA-sequences and polypeptides.

FIG. 1

0 273 889

**Description**

Heteromultimeric proteins and their manufacture.

The present invention relates to a method of in vivo production of a heteromultimeric protein, the subunits of which together but not separately are capable of generating a specific bioactivity. the invention furthermore provides for a eucaryotic plasmid vector having introduced therein procaryotic DNA-constructs coding for the separate expression of the subunits of such heteromultimeric protein. Furthermore, the invention covers also cells harbouring such vector.

The present invention thus in general deals with the in vivo production of heteromultimeric proteins, such as heterodimeric proteins. Although the invention will be further exemplified with reference to heterodimeric proteins capable of generating bioluminescence, particularly the heterodimer composed of Luxα and Luxβ polypeptide subunits, it is to be observed that the invention is not in any manner limited to such expression systems but is generally applicable to any expression system capable of producing heteromultimeric proteins showing bioactivity.

## BACKGROUND OF THE INVENTION

Genes involved in bioluminescence have recently been isolated and expressed in Escherichia coli. The best characterized genes are the related luciferase luxAB genes from Vibrio harveyi, V. fisheri (1,2) and a non-homologous gene from the firefly P. pyralis (3). (Bracketed figures refer to the appended list of references the disclosures of which are incorporated herein by reference.)

The V. harveyi luciferase is a heterodimer, composed of Luxα and Luxβ polypeptide subunits (4), which catalyzes the oxidation of long chain fatty aldehydes. the reaction requires reduced flavin mononucleotide and molecular oxygen and results in the emission of blue-green (max 490 nm) light (5). Isolated luxA and luxB genes encode the α and β subunits of luciferase in E.coli. The expression of these polypeptides is sufficient to produce a functional luciferase enzyme, while the separate polypeptides do not emit light. E.coli cultures expressing luciferase enzyme are bioluminescent when the aldehyde substrate n-decanal is supplied, indicating that viable cells take up the aldehyde (1,6), and that reduced flavine mononucleotide and oxygen in adequate concentrations are intracellular avialable.

Previous work has demonstrated that a number of bacterial enzymes are expressed and can be used as selectable or detectable markers in transgenic plants, e.g. neomycinphosphotransferase (NTP-II) (7-9), chloroamphenicol-acetyltransferase (7), β-galactosidase (10) and hygromycin-phosphotransferase (HPT) (11).

All of the above-mentioned enzymes require relatively complex assay procedures and the accumulation of comparatively high amounts of gene product in the eucaryotic cells before detection can be accomplished. The results of CAT or β-galactosidase assays are not easily quantified, due to non-specific reactions or to the presence of endogenous enzyme activities in such cells. In addition, the cells must be destroyed to detect the presence of these enzymes, a fact which does not permit the continuous monitoring of gene expression during the development of the regenerating cells.

## SUMMARY OF THE INVENTION

To overcome the limitations associated with the prior art the invention is based on the use of a eucaryotic expression system for the production of heteromultimeric protein, the invention being exemplified in the following by using light emitting bacterial luciferase as a marker system for plant cell transformation. By measuring light emission, luciferase can be easily assayed and luciferase is, therefore, useful as a reporter gene in the transformation of eucaryotic cells.

To the best of our knowledge all the bacterial enzymes shown to be expressed in eucaryotic cells thus far have been of a single subunit type. The heterodimeric V.harveyi luciferase is a practically useful system to test for the assembly of complex bacterial enzymes in eucaryotic cells, such as plant cells, thus providing a pathway for the expression of multicomponent, heterologous enzyme systems in higher organisms, such as yeasts, plants and animals.

Accordingly, the invention provides a method of in vivo production of a heteromultimeric protein, the subunits of which together but not separately are capable of generating a specific bioactivity, such as bioluminescence. The method of the invention comprises the steps:

a) isolating at least two procaryotic genes coding for separate polypeptides;

b) eliminating all ATG:s on the 5′ end of each gene except the one coding for the starting methionine;

c) cloning the genes resulting from step b) into at least one eucaryotic expression vector;

d) introducing the expression vector(s) from step c) into a eucaryotic cell; and

e) allowing said cell to express separately but simultaneously and coordinately the protein subunits coded for by the said genes resulting in generation of said specific bioactivity.

The cloning of the step c) above can be performed either by cloning each gene into a separate eucaryotic expression vector or cloning all genes into one eucaryotic expression vector. In the former case all vectors must, of course, be capable of coexistence in a eucaryotic cell.

The invention is particularly applicable to the use of genes coding for the expression of protein subunits which together are capable of generating enzymatic activity. Said genes may code for the expression of a heterodimeric protein, such as V. harveyi luciferase. In a particularly preferred embodiment of the invention said genes code for the expres sion of the polypeptide subunits LuxA and LuxB.

The cloning of step c) is according to the invention performed on eucaryotic expression vectors, such as an animal vector, for example a mammalian expression vector, or a plant vector. Said genes are preferably of bacterial origin, but can also originate from other procaryots, such as blue-green algae.

In the technique of this invention the 5' ends provided under step b) above are suitably selected so as to be recognised by the translational system of the host cell.

A preferred embodiment of the method of the present invention enabling in vivo production of a functional heterodimeric protein in a eucaryotic cell thus comprises the following steps:

    a) isolating the structural genes of V.harveyi luciferase luxAB;

    b) constructing an aggregate of DNA-linker, single ATG-methionine start codon, and structural codon gene for each gene;

    c) cloning the aggregates resulting from step b) into a eucaryotic expression vector;

    d) introducing the expression vector resulting from step c) into a eucaryotic cell; and

    e) allowing said cell to express separately but simultaneously and coordinately the protein subunits LuxA and LuxB resulting in luciferase-mediated light emission from said cell.

According to another aspect of the invention there is provided a eucaryotic plasmid vector wherein there has been introduced procaryotic DNA-constructs coding for the separate expression of the subunits of a heteromultimeric protein capable of generating a specific bioactivity in vivo. Such activity may be of the bioluminescence type, such as that produced by V.harveyi luciferase.

According to a further aspect of the invention there are provided eucaryotic cells harbouring eucaryotic plasmid vectors as defined above. The invention also covers DNA-se quences and polypeptides as will be illustrated in the following disclosure.

The invention makes it possible for the first time to product heteromultimeric proteins of procaryotic origin in eucaryotic cells. Among all procaryotic proteins a considerable proportion is heteromultimeric, e.g. a number of very important enzymes. The production of procaryotic proteins in eucaryotic cells has many advantages:

    a) Many proteins are modified after translation (posttranslational modification and processing). This modification and processing is partly species specific and makes the proteins more acceptable to the organism and/or activates the proteins

    b) Procaryotic organisms have tough cell walls making it difficult to relase proteins without inactivation of their biological activity.

    c) Procaryotic cells and their growth media often contain immunogenic or otherwise harmful substances (e.g. enterotoxins or endotoxins), which may be difficult or impossible to remove by reasonable purification procedures. This is often a major problem with production of pharmaceuticals to be injected.

The preferred application of the invention using luxA and luxB gene cassettes to produce bacterial luciferase in eucaryotic cells has many analytical applications. These applications are based on the possibility to obtain emission of light (bioluminescence) from cells containing bacterial luciferase. The intensity of the emitted light is related to intracellular levels of bacterial luciferase and other components participating in the bacterial luciferase reaction (certain long chain aldehydes, reduced flavin mononucleotide and oxygen). Provided that concentrations of all except one component of the bacterial luciferase reaction are known (or constant) the measurement of light emission is a very convenient and rapid in vivo measurement of the unknown component. Since cell walls don't have to be opened the measurement is non-destructive. Furthermore it is rapid, extremely sensitive, continuous (rapid changes may be monitored) and requires only simple equipment (a luminometer only consists of a light detector, e.g. a photomultiplier, a photodiod or a photographic film, enclosed in a light tight chamber). At a constant bacterial luciferase level analytical conditions may be arranged for measurement of intracellular levels of e.g. certain aldehydes, oxygen or reduced flavin mononucleotide. Measurement of the latter would give a measure of the energy or reduction-oxidation level of the intracellular compartment.

The kind of measurement indicated above cannot be done with non-bioluminescent technique. Other techniques are either much less sensitive (several orders of magnitude) or require opening of cells walls to release the analyte. Opening of cell walls is not only a destructive procedure but may also introduce additional sources of error. Furthermore the resolution in time with such procedures would not allow monitoring of rapid changes. In eucaryotes the use of lux genes to monitor intracellular events at the gene, protein of physiological level represents a considerable improvement as compared to known techniques (the technique has been used already in procaryotes). Commercially interesting applications include:

a) Mutagenicity testing.

LuxA and B gene cassettes containing a mutation resulting in a non-luminescent bacterial luciferase can in princip be inserted in the genom of any cell. In the presence of mutagens such cells would change from a non-luminescent to a luminescent form, a change that could easily be continuously and automatically monitored in a suitable luminometer (already available on market). With known techniques mutagenicity testing is done using mutagenized bacteria unable to grow on certain media. Mutation results in bacterial cells able to grow on these media. Growing (mutant cells can be detected as colonies after over-night incubation.

This so called Ames test involves several disadvanta ges. Firstly, the technique is performed with bacterial cells with genetic processes somewhat different from the eucaryotic cells that are of interest in most mutagenicity testing, i.e. human cells. Secondly, in the human organism mutagenes are metabolized to other substances that often are more mutagenic than the original substance (to some extent this problem is counteracted by inclusion of extracts from mammalian tissues in the incubation of bacterial cells with mutagens). Thirdly, the Ames test is unsuited for automation and requires overnight incubation. The disadvantages of the Ames test make it necessary to confirm results in animal experiments. Also known today is a mutagenicity test involving dark mutants of luminescent bacteria (e.g. B.Z.Levi, J.C. Kuhn and S. Ulitzur, Mutat.Res. 1986 April, 173(4):233-7). This technique is several orders of magnitude more sensitive and much more convenient than the Ames procedure, but suffers from the same first two disadvantages stated above for the Ames test. A mutagenicity testing according to the present invention could not only be performed with human cells but with exactly the cells that are of interest in a particular situation. The susceptibility of e.g. a certain type of lung cells to a potential mutagen can be studied in a particular patient.

Possible products would be mutants of luxA and luxB genes (with and without vectors and promoters for various types of cells). The market would be clinical and genetic research institutes and chemical companies involved in mutagenicity testing and, in the future, routine clinical laboratories.

b) Therapeutic monitoring of agents affecting cell growth.

If a drug (e.g. an anti-cancer drug) acts by inhibiting the growth of a certain cell type this effect can be studied by inserting the lux genes into the cells of interest (e.g. cancer cells from a certain patient) followed by measurement of light emission during growth conditions in the presence of various anti-cancer drugs. This can be used for finding the drug most efficient in retarding growth, most likely also being the most efficient anti-cancer drug. Inversely a deficiency for a certain growth factor in a cell can be found by inserting the lux genes into the genom conveniently measuring growth as light emission in the presence of various potential growth factors. The measuring technique in these types of applications can be easily automated and may well develop into routine clinical assays.

Possible products would be luxA and luxB cassettes (with and without vectors and promoters for various types of cells) and special cell lines containing these genes. The market would be clinical research institutes, pharmaceutical companies and, in the future, routine clinical laboratories.

c) Monitoring of gene transfer between cells and gene expression under various conditions.

When transferring a gene between cells the gene is often associated with an indicator gene in the same (or in an identical) vector. The indicator gene is used for selection of cells that have accepted the vector. The success of a transfer of a gene is usually confirmed by assaying the appearance of product in the recipient cell. This is often a complicated procedure and requires a lot of time. Furthermore, conditions may not be right for gene expression although the gene has actually been transferred. According to the invention the gene that is to be transferred is inserted in a vector containing the lux genes and under control of an identical promoter. Thus transfer and expression of gene can be monitored by measuring the light emission. This procedure is convenient, rapid and sensitive. The usefulness of this approach has been shown for lux genes transferred between bacterial strains (J. Engebrecht, M.Simon and M.Silverman, Science 1985 March 15; 227(4692):1345-7). According to the present invention this can now be done with eucaryotic cells. The technique is not limited to transfer of procaryotic genes, but vectors carrying the lux genes as indicator genes can be used to transfer any gene in any type of cell provided suitable vectors and promoters are used.

The technique can also be used for monitoring cell fusion resulting in hydridoma cells. Monoclonal antibodies are produced by hybridomas between rapidly growing myeloma cells and antibody producing B-cells not suited for growth. Labelling e.g. myeloma cells with luxA gene and B-cells with luxB gene would give a convenient way of monitoring hybridoma formation by measuring onset of light emission.

Possible products in these types of applications would be luxA and luxB cassettes (with and without suitable vectors and promoters for various cell types) and cell lines containing these genes.

DESCRIPTION OF SPECIFIC EMBODIMENTS

The invention will now be further exemplified by specific, non-limiting embodiments, and the illustration of the invention will be made under reference to the appended drawings.

Brief description of the drawings

Figure 1.

Construction and cloning of V.harveyi luciferase luxA and luxB gene cassettes in plant expression vector pPCV701

Construction of plant vector pPCV701luxA&B, carrying luxA and luxB genes under transcriptional control of $T_R$DNA promoters 1' and 2' is described in Example 1 Materials and Methods.

(P1BLA)-P1 promoter of $\beta$-lactamase gene (P1', P2')-promoters of $T_R$-DNA encoded genes 1' and 2', (P$_{NOS}$) nopaline synthase promoter, (Pg5)-promoter of $T_L$-DNA) encoded gene 5, (g4pA), (OcspA), (g7pA)-polyadenylation sequences derived from TL-DNA encoded gene 4, the octopine synthase gene and gene 7;

(NPT-II)-neomycin phosphotransferase gene, $(B_L)$, (BR)-25 bp left and right border repeats of T-DNA, (ori$_T$), (ori$_V$)-replication and conjugational transfer origin sequences derived from plasmid RK2; (ori pBR)-replication origin of pBR322; (A)ApaI, (B)BamHI, (Bg)Bg1II, (Bs)BstEII, (H)HindIII, (K)KpnI, (M)MaeI, (P)PstI, (Pv)PvuII, (R)EcoRI, (S)SalI, (X)XhoI, (Ss)SstII, (Sp)SspI.

Figure 2.

## Effect of plant extracts on luciferase activity

The relationship between light emission and amount of luciferase in the presence and absence of plant homogenates was determined by using commercially available V.harveyi luciferase.

In Fig. A, the filled squares indicate light units of luciferase activity in lux assay buffer. Open circles are light units of luciferase activity in lux assay buffer containing 0.1% bovine serum albumin (BSA). The closed circles are light units of luciferase activity in lux assay buffer in the absence of reduced FMN. Fig. B: Luciferase was mixed with N.tabacum SR-1 leaf extract, prepared by grinding in liquid nitrogen 1.0 g of leaf tissue in 4.0 ml lux assay buffer (50 mM $Na_2HPO_4$, pH7 containing 50 mM β-mercaptoethanol and 0.4 mM sucrose). Aliquots of the extract (0.5 ml) were assayed for luciferase activity (open circles). The closed circles indicate light units of luciferase activity in the absence of reduced FMN. Fig. C: Luciferase was mixed with carrot cell extracts ($10^7$ cells/ml), prepared as described in Materials and Methods and assayed for luciferase activity (open circles). The closed circles indicate light units of luciferase activity in the carrot extract in the absence of reduced FMN.

Figure 3.

## Representative time course of the bioluminescence reaction in trasnformed carrot protoplasts 24 hours after electroporation with plasmid pPCV701luxA&B DNA.

The substrate n-decanal and reduced FMN were injected into the extract at time zero. A similar time course of bioluminescence can be observed with any extracts prepared from pPCV701luxA&B transformed plant tissues by using assay conditions described in Materials and Methods. The initial maximum of light intensity is a measure of the initial velocity of luciferase reaction.

Figure 4.

## Immunoblot analysis of Luxα and Luxβ polypeptides in transformed carrot cells

Lanes 1 and 2 are extracts equivalent to 2 and $4\times10^6$ carrot protoplasts transformed by electroporation with pPCV701luxA&B DNA. Lane 3 = protein extract obtained from $4\times10^6$ untransformed control carrot protoplasts. Lane 4 = 10μg of commercial V.harveyi luciferase. Arrows indicated the position of Luxα and Luxβ polypeptides in transformed carrot protoplasts, lanes 1 and 2, and in the positive control, lane 4. No bands corresponding to Luxα and Luxβ were detected in the untransformed carrot protoplast extract, lane 3.

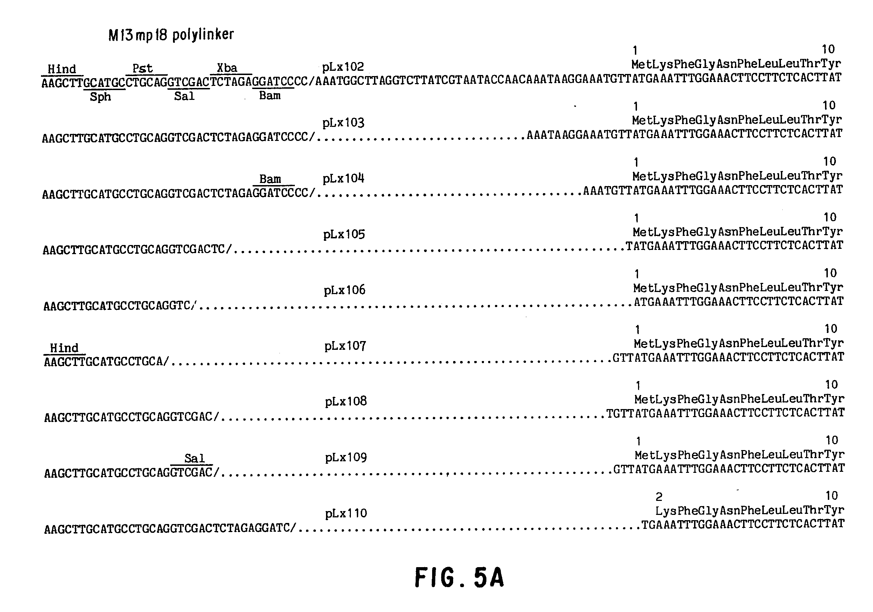

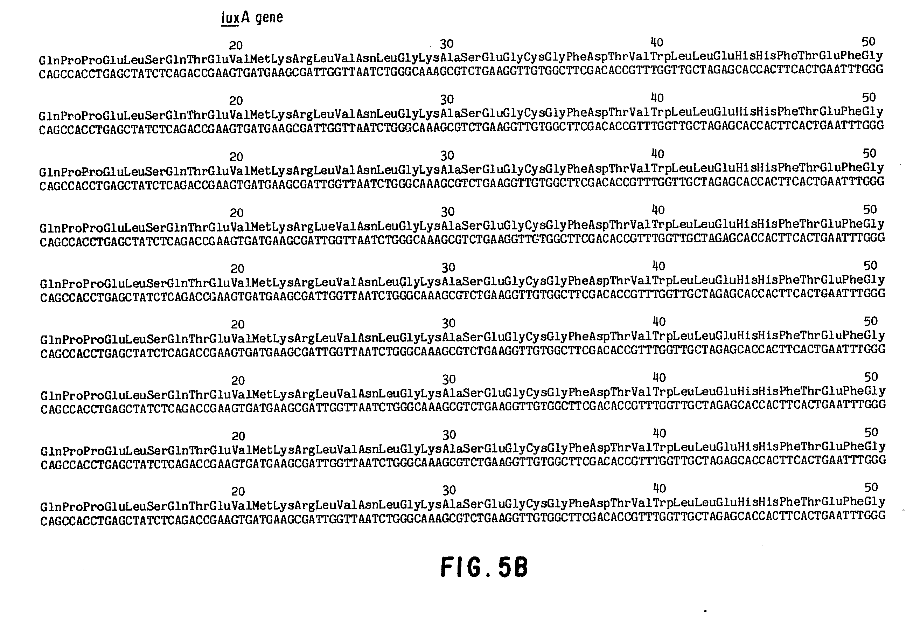

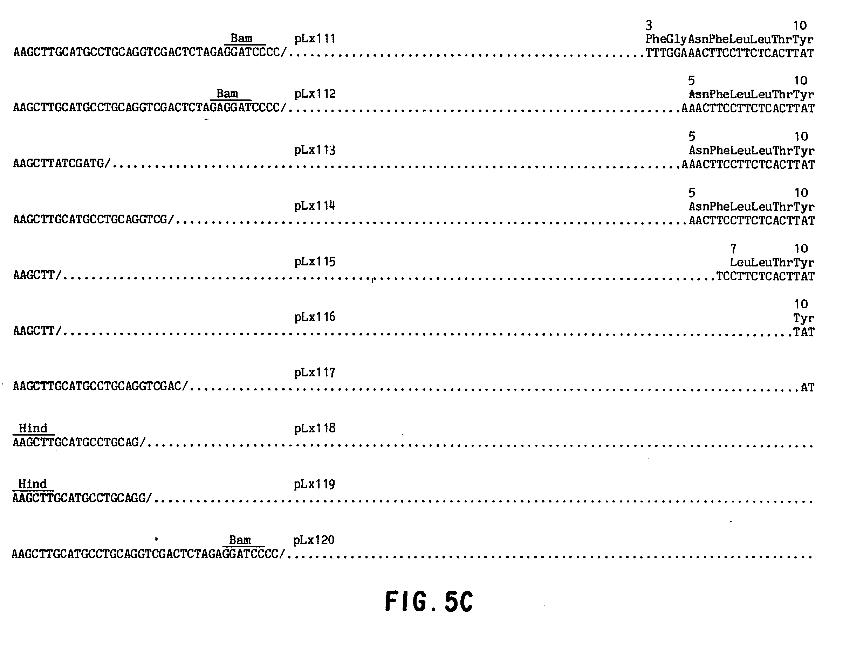

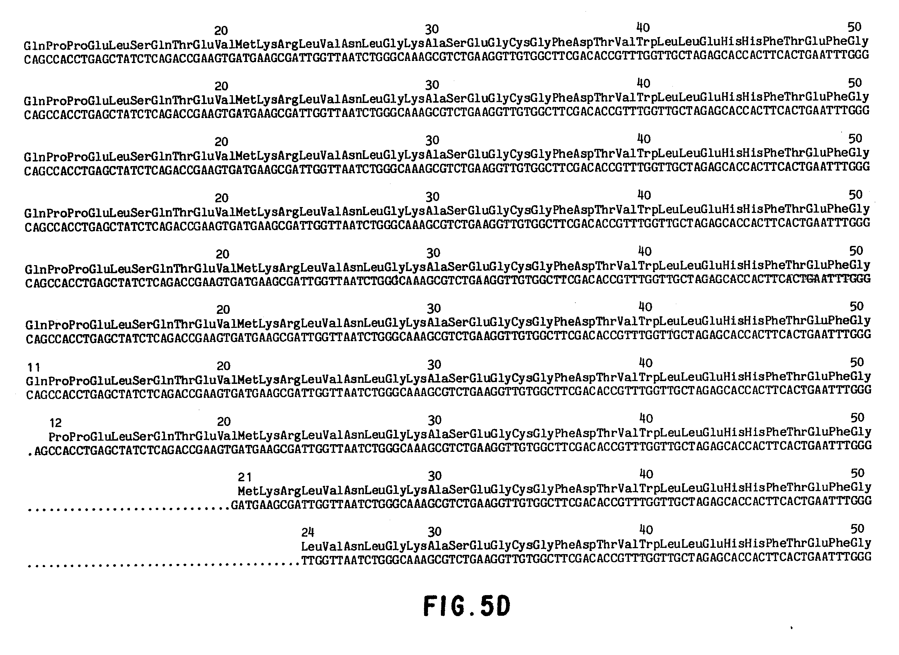

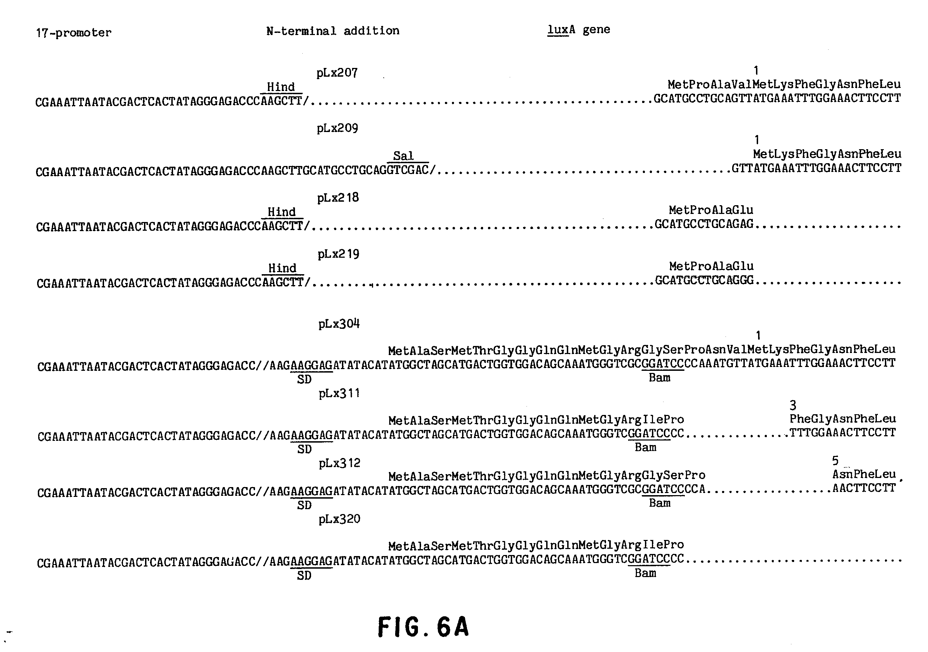

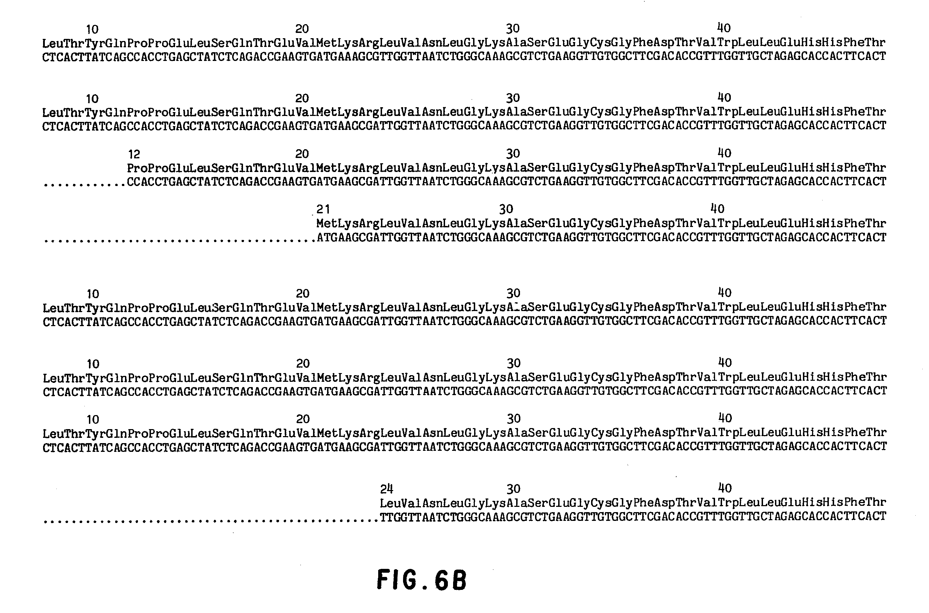

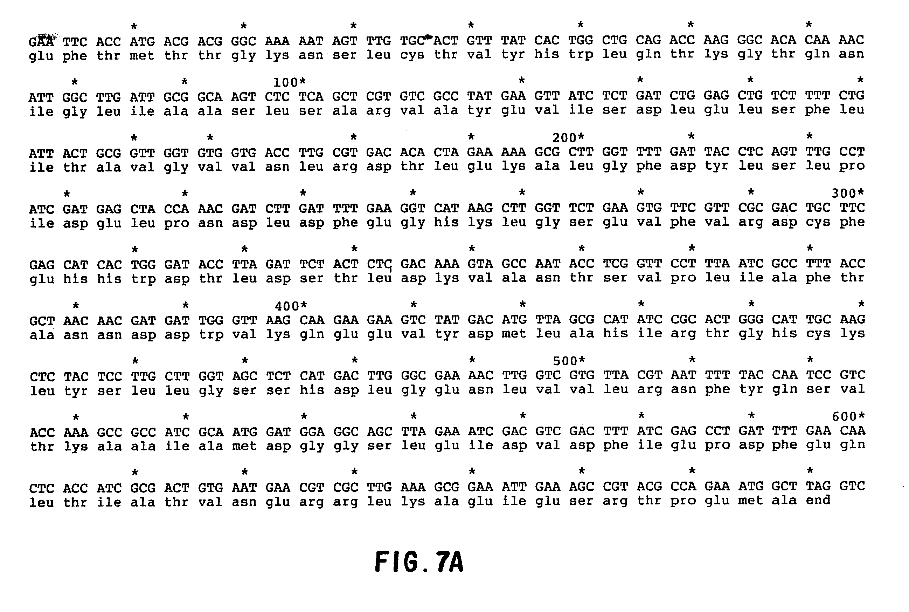

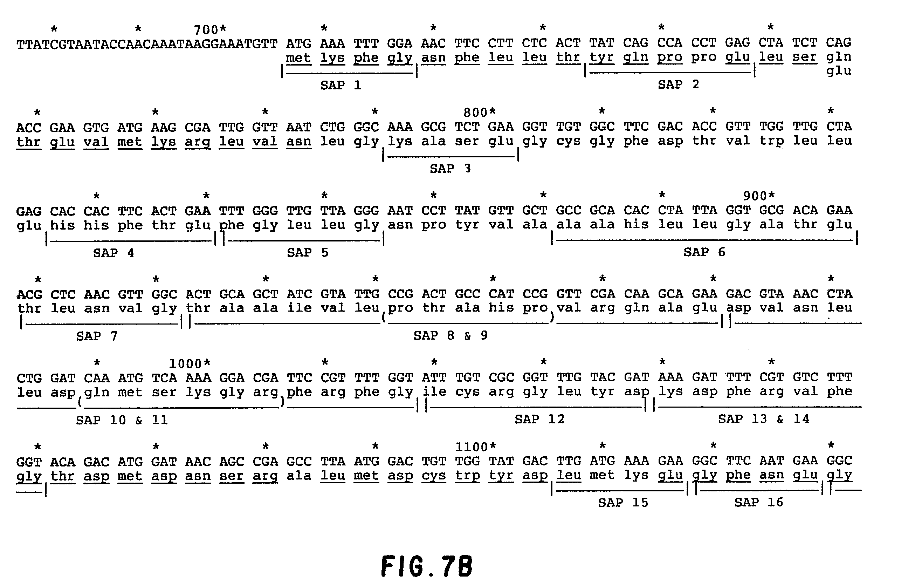

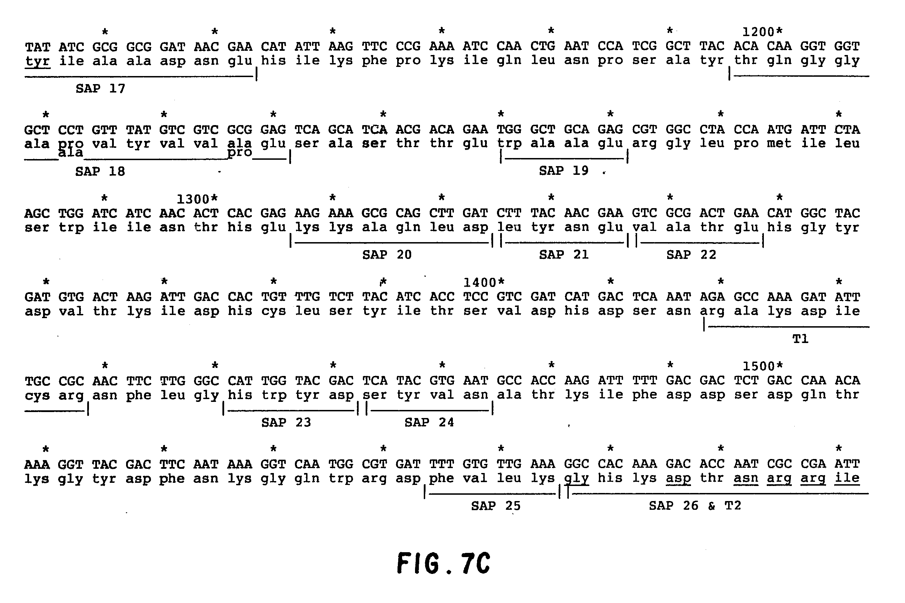

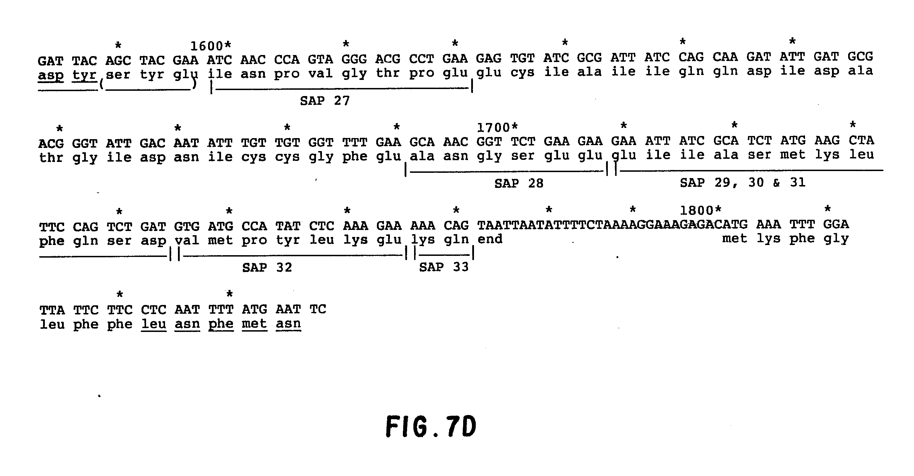

Figures 5 and 6 show the nucleotide sequence of the translated luxA gene and the corresponding polypeptides, the remaining C-terminal part being known per se (21) (copy of the relevant page being enclosed hereto as Fig. 7)

## EXAMPLE 1

## MATERIALS AND METHODS

### Cloning methods

Bacterial culture media, conditions for transformation of E.coli competent cells, procedures involving DNA fragment isolation, flushing of protruding 3' and 5' ends of DNA fragments by Klenow fragment of E.coli DNA polymerase I, T4 DNA polymerase of mung bean nuclease and for phosphatase treatment of DNAs, ligations and addition of synthetic oligonucleotide linkers were as described (13,14)

### Construction of plant expression vector pPCV701

Expression vector pPCV701 is an Agrobacterium binary plant cloning vector derived by a series of modifications from the plant vector pPCV002 described previously (15). Part of the vector pPCV701 extending from the ori$_V$ and ori$_T$ regions to the right 25 bp border sequence (Bp) remained identical to that of pPCV002. The plant selectable marker casset te from pPCV002, however, was modified by coupling the NPT-II coding sequence from the Bc1I-SmaI fragment of plasmid pKm 9 (16) to the promoter sequences of the nopaline synthase gene (8) and by adding the 3'-polyadenylation sequence of the $T_L$-DNA gene 4 (17; sequence between position 8840-9240 of the $T_L$-DNA). This selectable marker cassette was inserted between the HindIII and Bc1I sites of pPCV002, which destroyed the latter site and resulted in plasmid pPCV002NKMA. An expression unit was assembled as follows: BamHi-HindIII fragment of plasmid pAP2034 (18) was replaced by that of plasmid pOP44392 (19) to obtain plasmid pAPTR1'2' in which the promoter of gene 2' is linked to the polyadenylation sequences of the T-DNA gene 7, derived from plasmid pAR2034, (19). After opening pAPTR1'2' DNA by SalI, end filling with T4-DNA-polymerase followed by HindIII digestion, the polyadenylation sequence of the T-DNA octopine synthase gene, from plasmid pAGV40 (8,17), was added as a PvuII-HindIII fragment downstream from the gene 1' promoter. This resulted in the regeneration of a single SalI site. The expression cassette was thereafter isolated as an EcoRI-HindIII fragment, and inserted into pPCV002NKMA, which gave the final expression vector pPCV701.

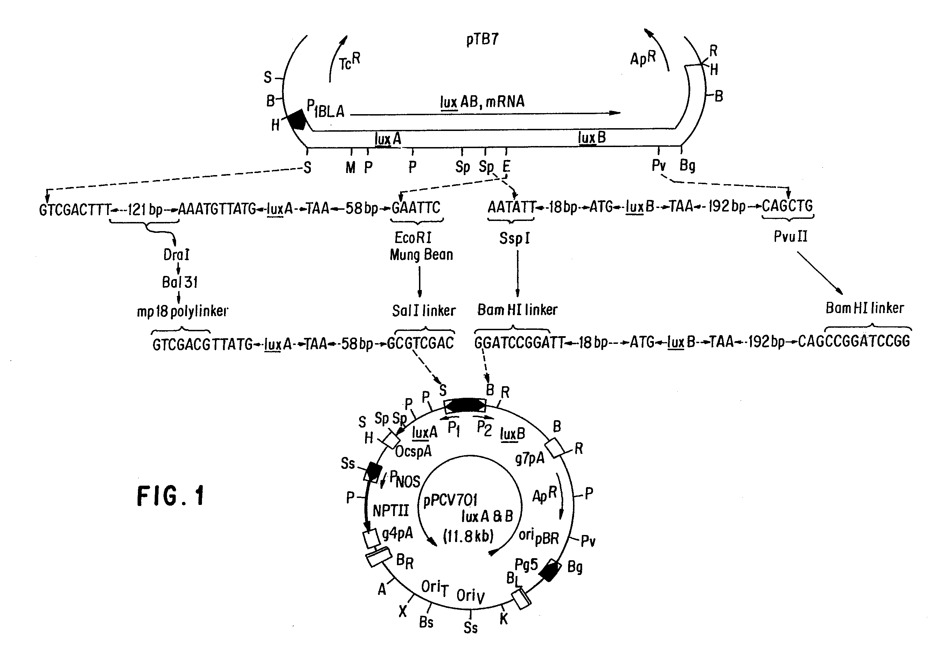

Construction and cloning of luxA and luxB gene cassettes in expression vector pPCV701 (Fig. 1).

Plasmid TB7, carrying the luxAB transcriptional unit, was linearized with SalI, and 10μg of the plasmid DNA was incubated in high salt buffer (100 mM Tris-HCl pH 8.0, 12 mM CaCl2, 600 mM NaCl, 1 mM EDTA) with increasing amounts (0.018-2.36 U) of Bal31 exonuclease at 30°C for 2-5 minutes, followed by EcoRI digestion. Fragments (1.0-1.2 kb in length) were isolated from gels after Bal31 digestion and subcloned into the DraI-EcoRI sites of pBR322 (20). Plasmid DNA prepared from pooled E.coli transformants recombinant plasmids were digested by DraI and EcoRI and the mixture of fragments was subcloned into pBR322 and enriched as described above. The ATG codon preceeding the translational initiation codon of the luxA protein is a part of an AAA triplet (21). Regeneration of DraI sites indicated that the endpoints of deletions were AAA triplets. Further digestion of DraI-EcoRI fragments isolated from the second pool of recombinants by MaeI showed that each possible deletion category starting from the SalI site of plasmid pTB7 was present in the pool. The DraI-EcoRI fragment pool was cloned into SamI-EcoRI sites of the M13 vector mp18 (22) and the exact endpoints of deletions were determined for 196 independent clones by DNA sequencing (23,24). One of the isolated deletion endpoints was located 7 bp upstream from the ATG initiation codon from luxA. This plasmid was opened at its BamHI site immediately upstream of the destroyed SmaI/DraI site, further deleted by Bal31 and then religated. The ligated samples were derived into three aliquots; one was digested by XbaI and the other by SalI, while the third sample was untreated. The samples were transfected into E.coli (24). Reduction in the number of plaques obtained with salI or XabI digestion of ligated samples as compared to that of the non-digested control indicated whether the Bal31 enzyme passed through these sites in the deletion reaction. Out of 98 clones sequenced, the extra ATG was removed from 8, and of these,4 retained the SalI site. M13 RF DNA was isolated from one of these clones, digested by EcoRI, the EcoRI sites were blunted by mung bean nuclease and ligated to SalI linkers. The resulting SalI linked constructions with all ATGs - except for the initiation ATG of the Luxα protein - removed, were sequenced with the -40 primer (Biolabs Cat. No. 1201) in one direction, and with the reverse sequencing primer (Biolabs Cat. No. 1212) in the opposite direction.

The luxB gene was isolated as an SspI-PvuII DNA fragment from the plasmid pTB7, followed by a BamHI linker addition to the 5' and the 3' ends, cloned in both orientations into the BamHI site of M13 mp18 and partially sequenced. The SalI luxA and the BamHI luxB cassettes were inserted in two steps into single SalI and BamHI sites of pPCV701, respecti vely. This resulted in plasmid pPCV701luxA&B and rendered luxA under gene 1' and luxB under gene 2' promoter control. pPCV701luxA&B was transformed into the E.coli strain SM10 and mobilized into Agrobacterium strain GV3101 (pMP90RK) as described (15).

Plant transformation and tissue culture

Agrobacterium strain GV3101 (pMP90RK) carrying plasmid pPCV701LuxA&B was used in protoplast co-cultivation (25,26) and plant tissue infection experiments (27) to transfer the luxA and luxB genes, as well as the NPT-II selectable marker gene into tobacco and carrot cells. Transformants were selected in the presence of 100 μg/ml kanamycin sulphate, and the Agrobacteria counter-selected by the addition of 500 μg/ml claforan (26). Conditions of tissue culture and tobacco plant regeneration were as described (15,25,26,28). Carrot protoplasts were isolated from cell line WOO1C by using 2.0% cellulase and 1.0% macerozyme according to Dudits (29). The protoplasts were resuspended in 30 ml of wash medium containing 0.37 M glucose,1.5mM CaCl2.H2O, and 10mM MES pH 6.5, counted, centrifuged and resuspended in protoplast wash medium at a final concentration of $1 \times 10^7$/ml. 1.0 ml aliquots were transferred to a multiwell culture plate (Falcon #3047) and plasmid pPCV701luxA&B DNA was added to each well to a final concentration of 50 μg/ml. The protoplast/SNA mixture was subjected to electroporation as described by Langridge et al. (30). The transformed protoplasts were resuspended in K-3 medium containing 0.4M sucrose as osmoticum and incubated at 26°C in the absence of light (29).

Luciferase assay

The activity of luciferase was measured by a luminometer (Turner TD-20e) as a means of total light produced during the first 10 seconds of enzymatic reaction (31). To calibrate each series of measurement a titration curve showing relationship between light emission and luciferase activity was established by measuring known amounts of commercially available V.harveyi luciferase (Sigma L-1637). Aliquots of 1:100 and 1:1000 dilutions of luciferase enzyme stock solution (1 mg/ml) were diluted with 0.5 ml lux assay buffer (50 mM sodium phopshate (pH7.0), 50 mM 2-mercaptoethanol) and transferred into the luminometer. The reaction was started by injection of a mixture of 0.5 ml 100μm reduced FMN and 10 μl n-decanal substrate through a septum in the top of luminometer sample chamber. The FMN solution was prepared in 25 mM EDTA (pH 7.0) or in 200 mM tricine buffer (pH 7.0) and kept reduced by light (32). The substrate was prepared as a 1:1000 dilution of n-decanal (Sigma D-7384) in lux assay buffer or in H2O and used freshly after sonication (33,34). At low protein concentrations the assay buffer was supplemented with 0.1% BSA. Linear titration plots were obtained in a concentration range of 1-50 ng luciferase/ml assay mix. It is important to note that, due to the inpurity of the preparation, the specific activity of commercially available luciferase was measured to approximately 100-fold less than that reported for purified V.harveyi luciferase enzyme (31).

6

Transformed tobacco tissues and carrot protoplasts and cells collected at different time intervals after electroporation were homogenized in 0.5-3.0 ml of lux assay buffer, centrifuged for 5 min in a Eppendorf centrifuge at 4°C. Aliquots of the cleared extracts were diluted in 0.5 ml assay buffer and their luciferase activity was measured as described below.

Both FMN reduction methods gave comparable luciferase activities in extracts of transformed cells. Pretreatment of plant protoplasts, cells and leaves, showed that luciferase activity remained unchanged when incubated in tricine containing buffer, but rapidly declined in the presence of EDTA. Therefore, to measure luciferase activities in intact plant tissue we used tricine to keep the flavin in the reduced state. When measuring luciferase in intact protoplasts, sucrose was included in the buffer to maintain the osmolarity of the solution at the same level as in the protoplast medium. The addition of sucrose to the assay buffer did not affect luciferase activity.

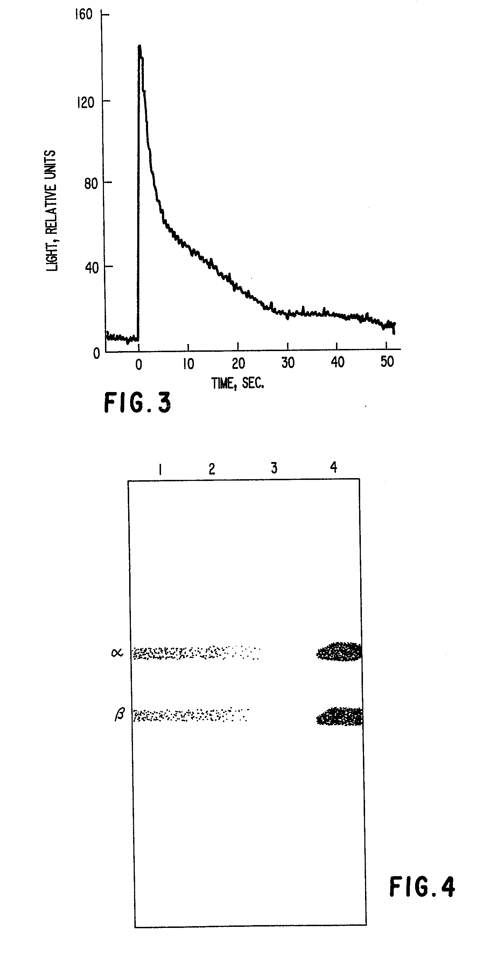

## Immunoblotting (Fig. 4)

The presence of Luxα and Luxβ polypeptides in transformed carrot protoplasts was detected by immunoblot analysis of proteins separated by SDS acrylamide gel electrophoresis. Protoplasts, incubated in culture medium after electroporation, were pelleted by centrifugation at 600 x g for 5 minutes at room temperature. The protoplast pellet was resuspended in 1.0 ml lux assay buffer, and luciferase activity was determined as described above. Total protein was precipitated with ethanol and incubation of the sample at -20°C for 1 hour. The precipitated protein was pelleted by centrifugation at 9.500 x g for 10 minutes at 4°C, resuspended in 100 μl of 2 times sample buffer, boiled for 2 minutes, and the proteins were separated on a 10% SDS polyacrylamide gel at 50 volts, for 12-14 hours (35). The separated proteins were transferred by Tobin et al. (36) and the blot was incubated at 26°C for 12 hours with anti Luxα and anti Luxβ IgG in a 40 ml volume of 10 mM Tris-HCl pH 7.4 containing 0.9% NaCl and 3.0% BSA. The luciferase specific IgG was removed by washing the filter 5 times in the above mentioned buffer, without BSA. The immunoblot was then transferred into 40 ml BSA containing buffer and incubated for 6 hours with goat anti-rabbit IgG conjugated to alkaline phosphatase. Following binding, the filter was washed several times to remove the excess amount of the second antibody, and the luciferase α and β polypeptides were identified by incubating the blot in 50 ml of 10 mM Tris-HCl, pH7.4, containing 30 μl of hydrogen peroxide and 30 mg of 4-chloro-1-naphtol.

## Results

## Conversion of luxAB transcriptional unit into separate "transcription-translation" cassettes

In the V.harveyi genome, the luxAB structural genes are part of a single transcriptional unit, encoding the α and β luciferase polypeptides of molecular weights 40 kD and 36 kD, respectively (1). In order to obtain expression and to permit correct translation of these genes in plant cells, it was necessary to separate both genes and to remove possible translation initiation codons located in their 5' untranslated leader sequences. Two separate "transcription-translation cassettes" were therefore constructed as outlined in Figure 1. In the reconstruction of the luxA gene, 133 nucleotides, containing three non-essential ATG codons, were deleted from the 5' leader sequence. The final construct resulted in a "luxA cassette" bordered by synthetic SalI sites. The SalI site on the 5' end originates from the M13 mp 18 polylinker sequence and is separated by 2 bp from the correct initiation codon. The added 3' SalI linker is located 58 bp downstream from the translational stop codon of the luxA gene. A similar construct was also assembled by adding either BamHI or HindIII linkers to both the 5' and 3' ends of the luxA gene (data not shown). Construction of the "luxB cassette" is also shown in Figure 1. The "luxB cassette" has a 5' BamHI site separated by 23 bp from the first native ATG triplet and a 3' BamHI site 197 bp downstream from the stop codon. Alternative "luxB cassette" were also constructed by ligating synthetic SalI of HindIII linkers, at both the 5' and 3' ends of the luxB gene.

Since in V.harveyi the luxAB genes are linked in one transcriptional unit, it was important to determine whether a functional luciferase could also be assembled when the individual submits were translated from two separate transcriptional units. To answer this question, the "luxA cassette" was inserted into a pBR322-derived expression vector and transcribed by an upstream T7 promoter (37). Similarly, the "luxB cassette" was inserted into a pACYC184 derivative and transcribed by an identical T7 promoter. E.coli colonies containing both plasmids in the same cell exhibited high luciferase activity (Olsson et al., in prep.). These experiments demonstrate that when the α and β subunits of luciferase are translated from two different mRNAs, they can assemble to form a functional luciferase enzyme in E.coli.

## Use of a dual promoter vector allowing simultaneous expression of luxA and luxB in transgenic plants

n order to transfer both luxA and luxB genes simultaneously into plant cells and to allow Luxα and Luxβ proteins to be expressed, a plant expression vector was constructed from elements of available expression and binary cloning vectors.

The constructed vector pPCV701 was derived from binary cloning vector pPCV002 described earlier (15). The "luxA cassette" was inserted into the single SalI site and the "luxB cassette" into the BamHI site of the expression vector, and thereby placed under the transcriptional control of the $T_R$-DNA 1' and 2' promoters, respectively (18). The resulting plasmid, designated pPCV701luxA&B, was mobilized from E.coli to Agrobacterium and transferred into tobacco and carrot cells by using protoplast co-cultivation and leaf disc infection methods (25,27). Plasmid pPCV701 luxA&B DNA was also used for transformation of tobacco and carrot protoplasts by electroporation (30).

Quantitative assay of luciferase in plant extracts

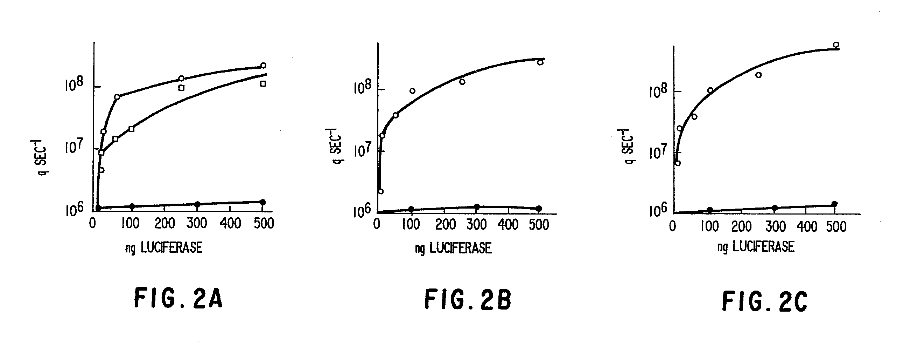

To determine if functional luciferase can be quantitatively assayed in plant extracts, known amounts of commercially available V.harveyi luciferase were mixed with carrot and tobacco cell extract and bioluminescence was measured. Figure 2 indicates that the light emission is proportional to known methods of luciferase in the presence or absence of plant extracts. Furthermore, as little as 0.5 ng of the com mercially available luciferase emitted 1.02 light units corresponding to $1.2 \times 10^6$ q sec $^{-1}$, clearly detectable in the assay. To check for the occurance of proteolytic degradation of luciferase enzyme in plant extracts, selected amounts of commercially avialable luciferase were mixed with extracts prepared from tobacco and carrot cells and incubated at 4°C for different time intervals. No proteolytic effect on luciferase was detected under these conditions. We therefore conclude that the values obtained for luciferase activity in the plant extracts represent an accurate estimate of the amount of luciferase protein present. When a standardized assay procedure is applied, it is possible to used bioluminescence as a quantitative and sensitive asay for the detection of luciferase activity in different plant extracts.

In order to determine how much, if any, luciferase activity could be contributed in various transformation experiments by Agrobacterium strain harbouring plasmid pPCV701-luxA&B, cell culture or sonicated cell extracts of this strain were assayed for luciferase activity (31). Luciferase activity was barely detectable, corresponding to less than 1.0 ng of luciferase/$10^6$ cells. In comparison, an E.coli strain carrying plasmid pTB7 (1), in which the luxAB operon is controlled by the P1 promoter of the pBR322 β-lactamase gene, produces 0.2 to 2.0 ng luciferase/$10^3$ cells (data not shown). The fact that luficerase expression was detected in Agrobacterium carrying plasmid pPCV701luxA&B was unexpected, as previously experimentation did not demonstrate expression of $T_R$-DNA 1' and 2' promoters in Agrobacterium. This result emphasizes the great sensitivity of the luciferase assay. In spite of low luciferase expression in Agrobacterium, particular care was taken to eliminate surviving Agrobacterium cells in transformed plant cultures prior to the luciferase activity measurements.

Demonstration of luciferase activity in transformed plant tissues

Results summarized in Table 1 demonstrate that luciferase activity can be easily and rapidly detected in transformed plant tissues. Due to the great sensitivity of the luciferase assay, the chimeric luxA&B genes can be used to demonstrate DNA uptake and gene expression in carrot protoplasts, as early as 8-24 hours after introduction of the DNA by electroporation.

As expected from the known properties of the bacterial enzyme, the activity of luciferase in plant extracts is also dependent on the addition of reduced flavin mononucleotide (FMN) and the long chain fatty aldehyde substrate n-decanal. Stable transformants in the form of carrot calli or tobacco plants emitted from 4.00 to 26.00 light units/gram wet weight of plant tissue, based on the luciferase assay (Table 1).

We found that luciferase activity can be accurately measured in intact protoplasts, as well as in plant homogenates (Table 1). When tricine was substituted for EDTA and 0.4% sucrose was added to the flavin solution to maintain the osmolarity, the majority of the protoplasts survived the assay procedure in good condition.

The expression of luciferase in transformed plant material requires the presence of both luxA and luxB products

Although the catalytic site for the luciferase activity is carried by the α-polypeptide, both Luxα and Luxβ polypeptides must be properly assembled in order to obtain light emission by E.coli cells or extracts (5). It is however conceivable that the α-polypeptide in plants might have independent luciferase activity as a result of an interaction with unknown plant factors. To rule out this possibility, we transformed carrot protoplasts with a plasmid construction designated pPCV701 luxA, which carries the correct promoter luxA gene fusion but not luxB gene fusion. No luciferase ac tivity was detected in transformed cells 24 hours or even 7 days after electroporation (Table 2). The converse experiment was performed in which carrot protoplasts were transformed by plasmid pPCV701luxB, a construction that carries only the correct promoter luxB gene fusion. As expected no luciferase activity was detected in the transformed cells (Table 2). However, when both luxA and luxB genes were present on the same plasmid high luciferase activity was measured in the electroporated carrot protoplasts. We therefore conclude that bioluminescence in transformed plant cells requires the simultaneous presence of both luxα and luxβ polypeptides. The fact that high light emission is obtained indicates that both polypeptides can assemble to a functional form in plant cells. As further and definitive confirmation that both Luxα and Luxβ polypeptides are present in transformed plant cells showing luciferase activity, extracts of carrot protoplasts, taken 34 hours after transformation with pPCV701 luxA&B DNA, were tested by immunoblot analysis. As is shown in Figure 4 transformed carrot protoplasts contained both luciferase subunits in similar amounts. By comparison, carrot protoplasts transformed with the luxA or the luxB construct were shown to contain only the expected subunits. The amount of the luciferase protein present in transfored protoplast extracts was estimated by comparison between measured light units and the light units emitted by a known amount of purified V.harveyi luciferase. It was reported that 1.0 mg of purified luciferase emits approximately $1.6 \times 10^{14}$ q sec $^{-1}$ light when n-decanal is used as substrate (34). Based on this value, the bioluminescence measured in $10^7$ carrot protoplasts (Table 1, A) was $5.4 \times 10^9$ q sec $^{-1}$, which

corresponds to 34 ng of luciferase. This amount of luciferase protein agrees well with the estimation from the intensity of the stained bands in the immunoblots.

Figure 1 shows how a dual promoter expression vector was used to separate the A and B cistrons of the V.harveyi luciferase operon into two separate plant transcription- translation units. After introduction of the luxA and luxB genes - carried by this dual promoter vector - into tobacco and carrot cells, it was possible to demonstrate luciferase activity by bioluminescence and the presence of the α and β subunits by immunoblotting. Both the luxA and the luxB genes were expressed simultaneously and to similar levels. Luciferase activity was detected only in plant cells carrying genes for both subunits thus excluding that the α subunit, which carries the catalytic site, would by itself be responsible for the observed luciferase activity. The results therefore indicate that the α and β subunits of the bacterial luciferase enzyme were properly assembled in plant cells.

In view of the fact that specific luciferase activity can easily and quantitatively be detected inplant cell extracts, as well as, in intact viable cells, this system appears to be ideally suited as a convenient reporter enzyme to monitor the transcriptional regulation of chimaeric genes in transgenic plants. Establishment of accurate in situ measurements of gene activity during embryogenic and organogenic development should now be possible in intact plants. Bacterial luciferase could also be an ideal reporter enzyme to quantitatively test various 5′ upstream sequences for transscription promoter activity in transient gene expression assays.

EXAMPLE 2

A SalI/EcoRI fragment from the plasmid pTB7 (1,21) was cloned into the M13 phage mp18. A series of N-terminal deletions were constructed by either Exonuclease III treatment followed by Mung bean nuclease digestion, or by phage Bal31 exonuclease digestions. After the exonuclease digestions, the phage DNAs were religated, alternatively the deleted pieces were recloned into mp18. All constructs were thereafter DNA sequenced over the 5′ region of the gene, to exactly determine were the deletion had occured.

Fig. 5 shows a representative number of the luxA N- terminal deletions in the mp18 phage, where all upstream restriction sites come from the mp18 polylinker sequence, and the luxA sequence ends by an EcoRI site about 60bp, downstream of the stop codon for translation of the luxA polypeptide.

The formed plasmids were denoted the prefix pLX followed by a number starting from 101. The series continues to pLX120, in which 69 bp were deleted, counting the A of the first methionine (ATG) of the LuxA polypeptide as +1.

The luxB gene was cloned separately into M13mp18 as a BamHI linkered fragment. This plasmid was denoted pLX150.

In order to investigate whether the different truncated LuxA polypeptides were still active when in complex with the LuxB polypeptide, a starting methionine ATG codon had to be engeneered in the correct reading frame, on to the 5′ end of the luxA gene to be tested. In some cases this was possible by using an upstream ATG in the mp18 polylinker sequence. This was accomplished by excising the gene by using a DNA restriction enzyme site up-stream of the in frame ATG in the polylinker sequence and by cloning the luxA N-terminus deleted DNA fragment into an T7 polymerase expression vector, excising the gene by using a DNA restriction enzyme site upstream of the in frame ATG in the polylinker sequence.

The different constructs were tested in the in vivo T7 polymerase expression system as described (43). As a T7 polymerase promoter source the plasmid vector pT3/T7-19 was sometimes used (BRL Cat No 5379SA). The luxA gene derivatives from the pLX100 plasmids were cloned into pT3/T7-19 as either a SalI/EcoRI or a HindIII/EcoRI fragment. In this way a new series of plasmids were constructed denoted pLX200 etc. (Table III, Fig. 6). Furthermore, in order to obtain luxA N-terminal translational fusions, a vector series denoted pAR3038, pAR3039 and pAR3040 was exploited (42). These vectors carry a BamHI site in all three reading frames situated 12 amino acids downstream of the starting methionine codon of the gene 10 protein of phage T7.

The luxA fragments emanating from the pLX100 plasmid cloned into these vectors formed the pLX300 series (Table III, Fig. 6).

In order to test the activity of the N-terminally deleted or N-terminally added luxA genes, the pLX200 and pLX300 plasmids were transferred into an E.coli strain carrying the T7 DNA polymerase gene on the chromosome under control of the lacUV5 promoter (43). The luxB gene was added in trans on the pLX550:3 plasmid (Table III). This plasmid carries the luxB gene under the T7 promoter on plasmid pACYC184. It was constructed as follows: the luxB structural gene was excised from pLX150 as a BamHI fragment, and cloned into BamHI-site of the T7 promoter plasmid pAR2463 (42). From the thus created plasmid pLX350:3 , a BglII/HindIII fragment containing the active luxB gene fused to the T7 promoter was cloned into the BamHI/HindIII sites of plasmid pACYC184, giving rise to pLX550:3.

Alternatively, when testing for luciferase activity, the luxB gene was cloned into the EcoRI site of the pLX200 or pLX300 plasmids, giving rise to pLX200150 and pLX300150 luxAB plasmids respectively, and in this way restoring the original luxAB operon.

Additional lux constructs that were tested for activity was C-terminal protein fusions to the luxA gene. These were also transformed into the T7 polymerase expression strain, and complemented with plasmid pLX550:3 in a similar fashion as above.

Analogously, an eleven amino acids C-terminal deletion of luxB (A BamHI/ClaI cloning of pLX150 into pT3.T7-18, giving rise to pLX250), was tested by adding in trans the plasmid pLX509:3, which is a pACYC184 derivative plasmid carrying luxA gene under control of the T7-promoter.

Finally, in order to fascilitate the further use of the luxAB operon or the luxA, luxB gene system, chosen representatives of constructs with or without the ribosomal binding site, with single starting ATG codons, or different deletions were cloned into other vectors, such as pBR322 and pUC18 or pUC19. In this case the plasmids were denoted pLX600 and pLX400 respectively (Table III).

A series of vectors carrying luxA and luxB genes have been constructed. This allows for the possibility to excise the luxA and luxB genes by a number of different restriction enzymes, enhancing the use of these genes as markers in gene regulation studies. The two luciferase structural genes cloned under separate promoters have also been expressed in the eucaryotic cell, demonstrating that the rather complex luciferase reaction works also in higher cells. Therefore, these genes can be used as markers both in eucaryotic and procaryotic cells. A number of different N-terminal deletion derivatives of the luxA gene have beem characterized. It is shown that the luciferase enzyme remains active both when the luxA N-terminal carries an extra stretch of 17 amino acids (pLX304) or when 11 aminoacids are deleted and four extra are added (pLX 218).

The significance of these data is threefold:

i) Transcriptional fusions of the luxA and luxB genes to virtually any promoter are now possible, with or without the inherent luxA ribosomal binding site present in the fusion. We have shown that the two luciferases structural genes work as well in trans on two different replicons, as they do when situated in an operon. We have also confirmed other studies that the luxα polypeptide along or the β polypeptide alone show absolutely no luciferase activity (46). It is therefore now possible to design experiments where e.g. luxA is placed under a constitutive promoter and luxB under a timely regulated, such as a developmentally regulated, promoter. Only when the β polypeptide subunit is synthesized will the cells become bioluminiscent. Alternatively, the two genes can be placed on different replicons in different cells or on a cell and a virus - only when they come together in a cell fusion, or when a virus infection occurs will the luciferase enzyme become active.

ii) The technique of this invention gives many possibilities for translational fusion experiments. Such experiments have been a very important tool in gene regulation studies, exploring the β-galactosides gene as the reporter gene. Since the luciferase enzyme can be assayed on viable cells both faster and with higher sensitivity than the β -galactosides, luciferase fusions offer a good alternative in these kind of experiments.

iii) They add a variety of new luciferase proteins structures for structure and function related analyses. Only a few mutants in the luxA gene have been isolated so far. Here we described a large number of precise amino acid alterations that could be exploited e.g. in X-ray crystallographic studies.

From mutant enzyme analysis and from chemical modification studies it is known that the luxAB polypeptide complex contains a single active site which resides primarily if not exclusively on the α subunit. The specific role for the β subunit is unknown, but it is absolutely required for bioluminescence activity. Partial amino acid sequence information from regions though to be associated with the active center has been obtained (41). By chemical modification and partial proteolysis studies it has been shown that a highly reactive sulhydryl group, though to reside in or near the flavin binding site is located close to a region that is highly sensitive to proteases (38,41). In the complete sequence, this position is thought to be at the cysteinyl residue at position 106. It is suggested by several authors that the β-subunit might contribute directly to the structure of the active center by binding to this region (40,44,45). The subunit also shows a very interesting distribution of charge over the molecule, with a clustring of basic residues surrounding the reactive cysteinyl residue (39).

Generally the luxA and luxB sequences are similar, and the luxB gene is thought to have arisen as a gene duplication of luxA (21,39). Both genes are similar in their N-terminal part, and the most hydrophobic part of both the genes and the polypeptides is in the aminoterminal third. Therefore, it would appear critical to delete or alter too much of these regions. However, our data show that it is possible to add 17 amino acids to the N-terminal part of the α-peptide with high remaining activity of the αβ-complex. Deletions of up to 20 amino acids is possible but this reduces luciferase activity significantly. Also with a deletion of 3 N-terminal amino acids, the luciferase activity is affected. If this is due to reduced, activity of the αβ-complex or to a decreased stability of the α-polypeptide is not known at present.

Furthermore, we have demonstrated that C-terminal additions or deletion on the α or β subunit affect activity very little. Therefore, the great benefits of the luciferase system as a marker in gene regulation experiments can be explored with an even greater versatility. Herein there are shown vector constructs useful for many different transcriptional and translational fusion experiments for use in both procaryotic and eucaryotic cells.

E.coli harbouring plasmid pPCV701 has been deposited in accordance with the Budapest Treaty with the Deutsche Sammlung von Mikroorganismen (DSM), Göttingen, BRD, under deposit number 3920.

References

1. Baldwin, T.O., Berends, T., Bunch, T.A., Holzman, T.F., Rausch, S.K., Shamansky, L., Treat, M.L. & Ziegler, M.M. (1984) Biochemistry 23, 3663-3667.

2. Engebrecht, J. & Silverman, M. (1984) Pro.Natl.Acad.Sci USA 81, 4154-4158.

3. De Wet, J.R., Wood. K.V., Helinski, D.R. & DeLuca, M. (1985) Proc.Natl.Acad.Sci. USA 82, 7870-7873.

4. Hastings, J.W. & Nealson, K.H. (1977) Ann.Rev.Microbiol. 51, 549-595.

5. Ziegler, M.M. & Baldwin, T.O. (1981) Curr.Top.Bioenerg. 12, 65-113.

6. Belas, R., Mileham, A., Cohn, D., Hilmen, M., Simon, M. & Silverman, M. (1982) Science 218, 791-793.

7. Herrera-Estrella, L., De Block, M., Messens, E., Hernalsteens, J.P., Van Montagu, M. & Schell, J. (1983) EMBO J. 2, 987-995.

8. Herrera-Estrella, L., Depicker, A., Van Montagu, M. & Schell, J. (1983) Nature 303, 209-213.

9. Fraley, R.T., Rogers, S.G., Horsch, R.B., Sanders, P.R., Flick, T.S., Adams, S.P., Bittner, M.L., Brand, L.A., Fink, C.L., Fry, J.S., Galluppi, G.R., Goldberg, S.B., Hoffmann, N.L. & Wo, S.C. (1983) Proc.Natl.Acad.Sci. USA 80, 4803-4806.

10. Helmer, G., Casabadan, M., Bevan, M., Kayes, L. & Chilton, M.-D. (1984) Biotechnology 1, 520-527.

11. Van den Elzen, P.J.M., Townsend, J., Lee, K.Y. & Bedbrook, J.R. (1985) Plant.Mol.Biol. 5, 299-302.

12. Legocki, R.P., Legocki, M., Baldwin, T.O. & Szalay, A.A. (1986) Proc.Natl.Acad,SciUSA in press.

13. Maniatis, T., Fritsch, E.F. & Sambrook, J. (1982) Molecular Cloning Laboratory Manual, Cold Spring Harbor Laboratory, Cold Spring Harbor.

14. Koncz, C., Kreuzaler, F., Kalman, Zs. & Schell, J. (1984) EMBO J 3, 1929-1937.

15. Koncz, C. & Schell, J. (1986) Molec.Gen.Genet. in press.

16. Reiss, B., Sprengel, R. & Schaller, H. (1984) EMBO J. 3, 3317-3322.

17. Gielen, J., De Beuckeleer, M., Seurinck, J., Deboeck, F., De Greve, H., Lemmers, M., Van Montagu, M. & Schell, J. (1984) EMBO J. 3, 835-846.

18. Velten, J. & Schell, J. (1985) Nucleic Acids Res. 13, 6981-6998.

19. Velten, J., Velten, L., Hain, R. & Schell, J. (1984) EMBO J. 12, 2723-2730.

20. Bolivar, F., Rodriquez, R., Greene, P.J., Betlach, M., Heynecker, H.L., Boyer, H.W., Crosa, J. & Falkow, S. (1977) Gene 2, 95-113.

21. Cohn, D.H., Mileham, A.J., Simon, M., Nealson, K.H., Rausch, S.K., Bonam, D. & Baldwin, T.O. (1985) J.Biol.Chem. 260, 6139-6146.

22. Norrander, J., Kempe, T, & Messing, J. (1983) Gene 26, 101-106.

23. Hong, G.F. (1981) Bioscience Rep. 1, 243-252.

24. Messing, J. (1983) Methods in Enzymology, eds.Wu, R., Grossman, L. & Moldave, K., Vol. 101, pp 20-78.

25. Marton, L., Wullems, G.J., Molendijk, L. & Schilperoort, R.A. (1979) Nature 277, 129-131.

26. De Block, M.,Herrera-Estrella, L., Van Montagu, M., Schell, J. & Zambryski, P. (1984) EMBO J. 3, 1681-1690.

27. Horsch, R.B., Fry, J.E., Hoffman, N.L., Eichholz, D., Rogers, S.G. & Fraley, R.T. (1985) Science 227, 1229-1231.

28. Vasil, I.K., (ed.) (1984) Cell culture and somatic cell genetics of plants, Vol.1 (Academic Press Inc.).

29. Dudits, D. (1984) in Cell Culture and Somatic Cell Genetics of plants, ed. Vasil, I.K. (Academic Press) Vol.1, pp. 391-397.

30. Langridge, W.H.R., Li, B.J, & Szalay, A.A. (1985) Plant Cell Rep. 4, 355-359.

31. Hastings, J.W., Baldwin, T.O. & Nicoli, M.Z. (1978) Methods in Enzymology, ed. De Luca, M.A., Vol. LVII, pp. 135-152 (Academic Press).

32. Nelson, N., Nelson, H. & Racker, E. (1972) Photochem. and Photo-biol. 16, 481-489.

33. Hastings, J.W. & Weber, G. (1963) J.Opt.Soc.Am. 53, 1410-1415.

34. Hastings, J.W. & Reynolds, G. (1966) in Bioluminescence in Progress, eds. Johnson, F.H. & Haneda, Y. (Princeton University Press) pp. 45-62.

35. Laemmli, U.K. (1970) Nature 227, 680-685.

36. Tobin, H., Staehelin, T. & Gordon, J. (1979) Proc.Natl.Acad.Sci. USA 76, 4350-4354.

37. Studier, F.W. & Moffatt, B.A. (1986) J.Mol.Biol. 189, 113-130.

38. Dougherty, J.J., Jr., Rausch, S.K. & Baldwin, T.O. (1982) in Flavins and Flavoproteins (Massey, V. and Williams, C.H., eds) pp 379-382, Elsevier/North-Holland, New York.

39. Johnston, T.C., Thompson, R.B. & Baldwin, T.O., J.Biol.Chem. In Press 1986.

40. Meighen. E.A. & Bartlet, I. (1980) J.Biol.Chem, 255, 1181-1186.

41. Rausch, S.K., Dougherty, J.J., Jr. & Baldwin, T.O. (1982). In Flavins and Flavoproteins (Massey, V. and Williams, C.H. eds) pp 375-378, Elservier/North-Holland, New York.

42. Rosenberg, A.H., Dunn, J.J. & Studier, W. (1986) Manus in prep.

43. Studier, F.W. & Moffatt, B.A. (1986), J.Mol.Biol. 188 pp---.

44. Tu, S.-C. & Henkin, J. (1983) Biochemistry 22, 519-523.

45. Welches, W.R. & Baldwin, T.O. (1981) Biochemistry 20, 512-517.

46. Ziegler, M.M. & Baldwin, T.O. (1981) Curr.Top.Bioenerg. 12, 65-113.

## TABLE I

### Assay of luciferase in transformed plant tissues

| | Carrot | | | | | Tobacco | |
|---|---|---|---|---|---|---|---|
| | A | B | C | D | E | F | G |
| Extract | 34.0 | 1.4 | 3.9 | 50.0 | 10.0 | 16.6 | 5.6 |
| Extract + n-de-canal | 32.0 | 1.7 | 1.4 | 50.0 | 10.0 | 16.6 | 10.4 |
| Extract + n-de-canal +reduced FMN | 4522.0; | 5516.0; | 1.2; | 26065.0; | 114.0; | 4152.0; | 9.6 |

A = carrot protoplasts extracted 24 hours after electroporation with plasmid pPCV701luxA&B DNA

B = intact carrot protoplasts measured 24 hours after electroporation in tricine buffer.

C = untransformed carrot protoplast homogenate (control).

D = Agrobacterium transformed carrot cell extract.

E = extract of non-transformed carrot cells supplemented with 100 ng of commercially available luciferase.

F = leaf extract of a tobacco plant transformed by Agrobacterium carrying plasmid pPCV701luxA&B.

G = leaf extract of a control untransformed N.Tabacum SR1 plant.

Luciferase activities are given in ligt units where 1LU = $1.2 \times 10^6$ quanta sec $^{-1}$. A, B and C extracts were prepared from $1 \times 10^7$ protoplasts. A and B represent luciferase activities in transient expression experiments with 50μg pPCV701-luxA&B DNA. D-G are 1 g wet weight equivalent tissue extracts. The luciferase assays were carried out as described in Materials and Methods. Values obtained by omitting reduced FMN from the assay mix indicate that the level of endogenous reduced FMN in tissue culture cells is not sufficient for in situ detection of luciferase activity followed n-decanal addition alone.

TABLE II

Simultaneous expression of luxA and luxB genes is required

for luciferase activity in plant cells

|  | Donor DNA | | |
|---|---|---|---|
| Assay conditions | A | B | C |
| Carrot protoplast homogenate | 5.2 | 3.1 | 17.0 |
| Carrot protoplast homogenate + n-decanal | 2.3 | 2.7 | 1.4 |
| Carrot protoplast homogenate + n-decanal + reduced FMN | 5.9 | 4.8 | 4280.0 |

Carrot W001C protoplasts were prepared and transformed as described in Materials and Methods. Values are expressed in light units/$10^7$ protoplasts, extracted 34 hours after electroporation with 50μg/ml. (A) plasmid pPCV701luxA, containing only the luxA gene cassette, (B) plasmid pPCV701LuxB, containing only the luxB gene cassette, and (C) = plasmid pPCV701luxA&B, containing both luxA and luxB gene cassettes.

TABLE III: LX plasmids

| | |
|---|---|
| pLX101 | Mp18 lux A Sal/Eco (from TB7) |
| pLX102 | Mp18 lux A + 45/Eco |
| oLX103 | Mp18 lux A + 15/Eco |
| pLX104 | Mp18 lux A +  7/Eco |
| pLX105 | Mp18 lux A 87/Eco |
| pLX106 | Mp18 lux A 77/Eco |
| pLX106:2 | pLX106 HIII linker-Eco site |
| pLX107 | Mp18 lux A 80/Eco |
| pLX108 | Mp18 lux A 81/Eco |
| pLX109 | Mp18 lux A 90/Eco |
| pLX109:2 | pLX109 HIII linker-Eco site |
| pLX109pM1 | C-term.del.lux A,N-term.del. luxB with primer |
| pLX109pM2 | C-term.del.(stop codon) N-term. luxB with primer |
| pLX110 | Mp18 lux A - 1/Eco |
| pLX111 | Mp18 lux A - 6/Eco |
| pLX112 | Mp18 lux A - 11:1/Eco |
| pLX113 | Mp18 lux A - 11:2/Eco |
| pLX114 | Mp18 lux A - 12/Eco |
| pLX115 | Mp18 lux A - 16/Eco |
| pLX116 | Mp18 lux A - 27/Eco |
| pLX117 | Mp18 lux A - 28/Eco |
| pLX 118 | Mp18 lux A - 28´/Eco |
| pLX119 | Mp18 lux A - 69/Eco |
| pLX120 | Mp18 lux A - 69´/Eco |
| pLX121 | Mp18 lux A 90:40 (Sal linker on 3´ site pLX109) |
| pLX150 | Mp18 lux B Bam/Bam 5´-3´ |
| pLX151 | Mp18 lux B Bam/Bam 3´-5´ |
| pLX207H | (H/E pLX107 to T3/T7-19) |
| pLX209S | (S/E pLX109 to    "     ) |
| pLX218H | (H/E pLX118 to    "     ) |
| pLX219H | (H/E pLX119 to    "     ) |
| pLX202H | (H/E pLX102 to    "     ) |

```
pLX203H      (H/E pLX103 to    "      )
pLX204H      (H/E pLX104 to    "      )
pLX250       (B/Cla p LX150 to pT3/T7-18)
pLX209:1     (Sal/Eco pLX109 PM1 to PT3/T7-19)
pLX209:2     (Sal/Eco pLX109 PM2 to PT3/T7-19)
pLX207150    (E/E luxB pLX150)
pLX209150    (         "         )
pLX218150    (         "         )
pLX219150    (         "         )
pLX202150    (         "         )
pLX203150    (         "         )
pLX204150    (         "         )
pLX304       (B/E pLX104 to pAR3040)
pLX311       (B/E pLX111 to pAR3038)
pLX312       (B/E pLX112 to pAR3040)
pLX320       (B/E pLX120 to pAR3038)
pLX309:2     (pLX109 HindII/Eco in pAR2463
             Eco/HindIII repaired
pLX309:3     (pLX109 Sal fill in pAR2463 Bam fill in)
pLX350:2     (pLX150 Bam in pAR2152 Bam)
pLX350:3     (pLX150 Bam in pAR2463 Bam)
pLX304150    (E/E lux B pLX150)
pLX311150    (         "         )
pLX312150    (         "         )
pLX320150    (         "         )
pLX550:3     (BglII/Hind pLX350:3 in pACYC184
             Bam/Hind)
pLX509:3     (BflII/Hind pLX309:3 in pACYC184
             Bam/Hind)
pLX609H      (pLX109 HindIII linker in pBR322)
pLX609B      (pLX109 Bam linker in pBR322)
pLX609S      (pLX109 Sal linker in pBR322)
pLX651H      (PvuII/Bgl fragment from pTB7+Hind III
             linker)
pLX651B      (              "              Bam linker)
pLX651S      (              "              Sal linker)
```

```
pLX709      (pLX209:1 fusion lux B from pLX150)
pLX710      (pLX209:2          "                    )
pLX750      (fusion SphI/Hind pLX107:1 in pLX250)
pLX751      (fusion SphI/Hind pLX109:1 in pLX250)
```

<u>Key</u>

```
100      Mp18, Mp19
200      pT3/T7-18, pT3/T7-19 (BRL; cat no
300      pAR2151, pAR2463, pAR3038, pAR3039, pAR3040,
         (Studier and Moffatt, 1986)
400      pUC18, pUC19 (
500      pACYC184 (
600      pBR322 (
700      fusion protein A/B, B/A (Olsson et al, 1986)
```

## Claims

1. A method of in vivo production of a heteromultimeric protein, the subunits of which together but not separately are capable of generating a specific bioactivity comprising the steps:
   a) isolating at least two procaryotic genes coding for separate polypeptides;
   b) eliminating all ATG:s on the 5′ end of each gene except the one coding for the starting methionine;
   c) cloning the genes resulting from step b) into at least one eucaryotic expression vector;
   d) introducing the expression vector(s) from step c) into a eucaryotic cell; and
   e) allowing said cell to express separately but simultaneously and coordinately the protein subunits coded for by the said genes resulting in generation of said specific bioactivity.

2. A method according to claim 1, wherein under step c) each gene is cloned into a separate eucaryotic expression vector, all vectors being capable of coexistence in a eucaryotic cell.

3. A method according to claim 1, wherein all genes are cloned into one eucaryotic expression vector.

4. A method according to any preceding claim, wherein said genes code for the expression of protein subunits which together are capable of generating enzymatic activity.

5. A method according to claim 4, wherein said genes code for the expression of a heterodimeric protein.

6. A method according to claim 5, wherein said genes code for the expression of a bacterial luciferase, such as V.harveyi.

7. A method according to claim 6, wherein said genes code for the expression of the polypeptide subunits LuxA and LuxB.

8. A method according to any preceding claim, wherein the cloning of step c) is performed on a plant expression vector.

9. A method according to any of claims 1 to 7, wherein the cloning of step c) is performed on an animal, such as a mammalian expression vector or a fungi, such as yeast.

10. A method according to any preceding claim, wherein said genes are of bacterial origin.

11. A method according to any preceding claim, wherein the 5′ ends provided in step b) are selected so as to be recognized by the translational system of the host cell.

12. A method of in vivo production of a functional heterodimeric protein in a eucaryotic cell, comprising the steps:
   a) isolating the structural genes of a bacterial luciferase luxAB;
   b) constructing an aggregate of DNA-linker, single ATG-methionine start codon and structural gene for each of the lux genes;

c) cloning the aggregates resulting from step b) into a eucaryotic expression vector;

d) introducing the expression vector resulting from step c) into a eucaryotic cell; and

e) allowing said cell to express separately but simultaneously and coordinately the protein subunits LuxA and LuxB resulting in luciferase-mediated light emission from said cell.

13. A method according to claim 12, wherein the heterodimeric protein is expressed in a plant cell.

14. A method according to claim 13, wherein the expression takes place in a tobacco or carrot cell.

15. A method according to claim 12, wherein the heterodimeric protein is expressed in an animal cell, such as a mammalian cell.

16. A method according to claim 15, wherein the cell is a fungus cell.

17. A eucaryotic plasmid vector having introduced therein procaryotic DNA-constructs coding for the separate expression of the subunits of a heteromultimeric protein capable of generating a specific bioactivity in vivo.

18. A vector according to claim 17, wherein said DNA-constructs code for the expression of a heterodimeric protein.

19. A vector according to claim 18, wherein said DNA-constructs code for the expression of a bacterial luciferase.

20. A vector according to claim 19, wherein said DNA-construct codes for the expression of the polypeptide subunits LuxA and LuxB.

21. A vector according to any of claims 17 to 20, which is a plant expression vector.

22. A vector according to any of claims 17 to 20, which is an animal expression vector.

23. A eucaryotic cell harbouring a eucaryotic plasmid vector according to any of claims 17 to 22.

24. A cell according to claim 23, which is a plant cell, such as a tobacco or carrot cell.

25. A cell according to claim 23, which is an animal cell.

26. A cell according to claim 23, which is a fungus cell.

27. The nucleotide-sequence shown in Figs. 5 and 6 of the drawings as defined herein.

28. The polypeptides shown in Figs. 5 and 6 of the drawings as defined herein.

GTCGACTTT←121 bp→AAATGTTATG→luxA→TAA←58bp→GAATTC

DraI
Bal31
mp18 polylinker

GTCGACGTTATG→luxA→TAA←58bp→GCGTCGAC

SalI linker

EcoRI
Mung Bean

AATATT←18bp→ATG→luxB→TAA←192bp→CAGCTG

SspI

PvuII

BamHI linker

GGATCCGGATT←18 bp→ATG→luxB→TAA←192bp→CAGCCGGGATCCGG

BamHI linker

pTB7

lux AB, mRNA

TcR

luxA

luxB

ApR

P1BLA

S B H   S M P P Sp Sp E   Pv Bg   R H B

pPCV701
luxA & B
(11.8 kb)

luxA & B

P1 P2 luxB

luxA

OcspA

g7pA

ApR

oripBR

oriV

g4pA

PNOS

NPTII

OriT

Pg5

BL

BR

BR

K

Ss

Bs

X

A

Oriv

OriT

Ss

S Sp Sp

H

P

S   P P

S   B   R

B

R

P

Pv

Bg

FIG. 1

FIG. 2A

FIG. 2B

FIG. 2C

FIG. 3

FIG. 4

M13 mp18 polylinker

```
                                                                                    1                    10
  Hind        Pst           Xba            pLx102                          MetLysPheGlyAsnPheLeuLeuThrTyr
AAGCTTGCATGCCTGCAGGTCGACTCTAGAGGATCCCC/AAATGGCTTAGGTCTTATCGTAATACCAACAAATAAGGAAATGTTATGAAATTTGGAAACTTCCTTCTCACTTAT
     Sph          Sal          Bam
                                                                                    1                    10
                                           pLx103                          MetLysPheGlyAsnPheLeuLeuThrTyr
AAGCTTGCATGCCTGCAGGTCGACTCTAGAGGATCCCC/...........................AAATAAGGAAATGTTATGAAATTTGGAAACTTCCTTCTCACTTAT

                                                                                    1                    10
                                  Bam      pLx104                          MetLysPheGlyAsnPheLeuLeuThrTyr
AAGCTTGCATGCCTGCAGGTCGACTCTAGAGGATCCCC/...........................AAATGTTATGAAATTTGGAAACTTCCTTCTCACTTAT

                                                                                    1                    10
                                           pLx105                          MetLysPheGlyAsnPheLeuLeuThrTyr
AAGCTTGCATGCCTGCAGGTCGACTC/.............................TATGAAATTTGGAAACTTCCTTCTCACTTAT

                                                                                    1                    10
                                           pLx106                          MetLysPheGlyAsnPheLeuLeuThrTyr
AAGCTTGCATGCCTGCAGGTC/...........................ATGAAATTTGGAAACTTCCTTCTCACTTAT

                                                                                    1                    10
  Hind                                     pLx107                          MetLysPheGlyAsnPheLeuLeuThrTyr
AAGCTTGCATGCCTGCA/........................GTTATGAAATTTGGAAACTTCCTTCTCACTTAT

                                                                                    1                    10
                                           pLx108                          MetLysPheGlyAsnPheLeuLeuThrTyr
AAGCTTGCATGCCTGCAGGTCGAC/..............................TGTTATGAAATTTGGAAACTTCCTTCTCACTTAT

                                                                                    1                    10
            Sal                            pLx109                          MetLysPheGlyAsnPheLeuLeuThrTyr
AAGCTTGCATGCCTGCAGGTCGAC/.............................GTTATGAAATTTGGAAACTTCCTTCTCACTTAT

                                                                                    2                    10
                                           pLx110                          LysPheGlyAsnPheLeuLeuThrTyr
AAGCTTGCATGCCTGCAGGTCGACTCTAGAGGATC/.............................TGAAATTTGGAAACTTCCTTCTCACTTAT
```

# FIG. 5A

luxA gene

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLueValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

FIG. 5B

0273889

```
                                                              3                    10
                              Bam      pLx111                 PheGlyAsnPheLeuLeuThrTyr
AAGCTTGCATGCCTGCAGGTCGACTCTAGAGGATCCCC/..........................................TTTGGAAACTTCCTTCTCACTTAT

                                                              5                    10
                              Bam      pLx112                 AsnPheLeuLeuThrTyr
AAGCTTGCATGCCTGCAGGTCGACTCTAGAGGATCCCC/..............................................AAACTTCCTTCTCACTTAT

                                                              5                    10
                                       pLx113                 AsnPheLeuLeuThrTyr
AAGCTTATCGATG/........................................................................AAACTTCCTTCTCACTTAT

                                                              5                    10
                                       pLx114                 AsnPheLeuLeuThrTyr
AAGCTTGCATGCCTGCAGGTCG/...............................................................AACTTCCTTCTCACTTAT

                                                              7                    10
                                       pLx115                 LeuLeuThrTyr
AAGCTT/.....................................................................................TCCTTCTCACTTAT

                                                                                   10
                                       pLx116                                      Tyr
AAGCTT/.......................................................................................TAT

                                       pLx117
AAGCTTGCATGCCTGCAGGTCGAC/...................................................................................AT

   Hind                                pLx118
AAGCTTGCATGCCTGCAG/.........................................................................................

   Hind                                pLx119
AAGCTTGCATGCCTGCAGG/........................................................................................

                          Bam          pLx120
AAGCTTGCATGCCTGCAGGTCGACTCTAGAGGATCCCC/.....................................................................
```

# FIG. 5C

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

GlnProProGluLeuSerGlnThrGluValMetLysArgLeuValAsnLeuGlyLysAlaSerGluGlyCysGlyPheAspThrValTrpLeuLeuGluHisHisPheThrGluPheGly
CAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

ProProGluLeuSerGlnThrGluValMetLysArgLeuValAsnLeuGlyLysAlaSerGluGlyCysGlyPheAspThrValTrpLeuLeuGluHisHisPheThrGluPheGly
.AGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACTGAATTTGGG

MetLysArgLeuValAsnLeuGlyLysAlaSerGluGlyCysGlyPheAspThrValTrpLeuLeuGluHisHisPheThrGluPheGly
............................GATGAAGCGATTGGTTAATCTGGGCAAAGCGTCTGAAGGTTGTGGCTTCGACACCGTTTGGTTGCTAGAGCACCACTTCACTGAATTTGGG

LeuValAsnLeuGlyLysAlaSerGluGlyCysGlyPheAspThrValTrpLeuLeuGluHisHisPheThrGluPheGly
.................................TTGGTTAATCTGGGCAAAGCGTCTGAAGGTTGTGGCTTCGACACCGTTTGGTTGCTAGAGCACCACTTCACTGAATTTGGG

# FIG.5D

17-promoter           N—terminal addition         luxA gene

```
                    pLx207                                                    1
                      Hind                                 MetProAlaValMetLysPheGlyAsnPheLeu
CGAAATTAATACGACTCACTATAGGGAGACCCAAGCTT/...............................................GCATGCCTGCAGTTATGAAATTTGGAAACTTCCTT

                    pLx209
                                                                             1
                        Sal                                     MetLysPheGlyAsnPheLeu
CGAAATTAATACGACTCACTATAGGGAGACCCAAGCTTGCATGCCTGCAGGTCGAC/..................................GTTATGAAATTTGGAAACTTCCTT

                    pLx218
                      Hind                                  MetProAlaGlu
CGAAATTAATACGACTCACTATAGGGAGACCCAAGCTT/...............................................GCATGCCTGCAGAG.....................

                    pLx219
                      Hind                                  MetProAlaGlu
CGAAATTAATACGACTCACTATAGGGAGACCCAAGCTT/.........,.....................................GCATGCCTGCAGGG.....................


                    pLx304                                                             1
                             MetAlaSerMetThrGlyGlyGlnGlnMetGlyArgGlySerProAsnValMetLysPheGlyAsnPheLeu
CGAAATTAATACGACTCACTATAGGGAGACC//AAGAAGGAGATATACATATGGCTAGCATGACTGGTGGACAGCAAATGGGTCGCGGATCCCCAAATGTTATGAAATTTGGAAACTTCCTT
                                  SD                                           Bam
                    pLx311
                                                                                       3
                             MetAlaSerMetThrGlyGlyGlnGlnMetGlyArgIlePro            PheGlyAsnPheLeu
CGAAATTAATACGACTCACTATAGGGAGACC//AAGAAGGAGATATACATATGGCTAGCATGACTGGTGGACAGCAAATGGGTCGGATCCCC...............TTTGGAAACTTCCTT
                                  SD                                           Bam
                    pLx312                                                             5
                             MetAlaSerMetThrGlyGlyGlnGlnMetGlyArgGlySerPro            AsnPheLeu
CGAAATTAATACGACTCACTATAGGGAGACC//AAGAAGGAGATATACATATGGCTAGCATGACTGGTGGACAGCAAATGGGTCGCGGATCCCCA.................AACTTCCTT
                                  SD                                           Bam
                    pLx320
                             MetAlaSerMetThrGlyGlyGlnGlnMetGlyArgIlePro
CGAAATTAATACGACTCACTATAGGGAGACC//AAGAAGGAGATATACATATGGCTAGCATGACTGGTGGACAGCAAATGGGTCGGATCCCC............................
                                  SD                                           Bam
```

# FIG. 6A

```
        10                            20                            30                            40
LeuThrTyrGlnProProGluLeuSerGlnThrGluValMetLysArgLeuValAsnLeuGlyLysAlaSerGluGlyCysGlyPheAspThrValTrpLeuLeuGluHisHisPheThr
CTCACTTATCAGCCACCTGAGCTATCTCAGACCGAAGTGATGAAAGCGTTGGTTAATCTGGGCAAAGCGTCTGAAGGTTGTGGCTTCGACACCGTTTGGTTGCTAGAGCACCACTTCACT


        10                            20                            30                            40
LeuThrTyrGlnProProGluLeuSerGlnThrGluValMetLysArgLeuValAsnLeuGlyLysAlaSerGluGlyCysGlyPheAspThrValTrpLeuLeuGluHisHisPheThr
CTCACTTATCAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACT

        12                            20                            30                            40
            ProProGluLeuSerGlnThrGluValMetLysArgLeuValAsnLeuGlyLysAlaSerGluGlyCysGlyPheAspThrValTrpLeuLeuGluHisHisPheThr
............CCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACT

                             21                            30                            40
                              MetLysArgLeuValAsnLeuGlyLysAlaSerGluGlyCysGlyPheAspThrValTrpLeuLeuGluHisHisPheThr
...............................ATGAAGCGATTGGTTAATCTGGGCAAAGCGTCTGAAGGTTGTGGCTTCGACACCGTTTGGTTGCTAGAGCACCACTTCACT


        10                            20                            30                            40
LeuThrTyrGlnProProGluLeuSerGlnThrGluValMetLysArgLeuValAsnLeuGlyLysAlaSerGluGlyCysGlyPheAspThrValTrpLeuLeuGluHisHisPheThr
CTCACTTATCAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACT


        10                            20                            30                            40
LeuThrTyrGlnProProGluLeuSerGlnThrGluValMetLysArgLeuValAsnLeuGlyLysAlaSerGluGlyCysGlyPheAspThrValTrpLeuLeuGluHisHisPheThr
CTCACTTATCAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACT

        10                            20                            30                            40
LeuThrTyrGlnProProGluLeuSerGlnThrGluValMetLysArgLeuValAsnLeuGlyLysAlaSerGluGlyCysGlyPheAspThrValTrpLeuLeuGluHisHisPheThr
CTCACTTATCAGCCACCTGAGCTATCTCAGACCGAAGTGATGAAGCGATTGGTTAATCTGGGCAAAGCGTCTGAAGGTTGTGGCTTCGACACCGTTTGGTTGCTAGAGCACCACTTCACT


                    24                            30                            40
                      LeuValAsnLeuGlyLysAlaSerGluGlyCysGlyPheAspThrValTrpLeuLeuGluHisHisPheThr
......................................TTGGTTAATCTGGGCAAAGCGTCTGAAGGTTGTGGCTTCGACACCGTTTGGTTGCTAGAGCACCACTTCACT
```

# FIG. 6B

```
          *           *           *           *           *           *           *
GAA TTC ACC ATG ACG ACG GGC AAA AAT AGT TTG TGC ACT GTT TAT CAC TGG CTG CAG ACC AAG GGC ACA CAA AAC
glu phe thr met thr thr gly lys asn ser leu cys thr val tyr his trp leu gln thr lys gly thr gln asn

    *           *           100*                      *           *           *           *
ATT GGC TTG ATT GCG GCA AGT CTC TCA GCT CGT GTC GCC TAT GAA GTT ATC TCT GAT CTG GAG CTG TCT TTT CTG
ile gly leu ile ala ala ser leu ser ala arg val ala tyr glu val ile ser asp leu glu leu ser phe leu

          *           *           *           *           200*          *           *
ATT ACT GCG GTT GGT GTG GTG ACC TTG CGT GAC ACA CTA GAA AAA GCG CTT GGT TTT GAT TAC CTC AGT TTG CCT
ile thr ala val gly val val asn leu arg asp thr leu glu lys ala leu gly phe asp tyr leu ser leu pro

    *           *           *           *           *           *           *           300*
ATC GAT GAG CTA CCA AAC GAT CTT GAT TTT GAA GGT CAT AAG CTT GGT TCT GAA GTG TTC GTT CGC GAC TGC TTC
ile asp glu leu pro asn asp leu asp phe glu gly his lys leu gly ser glu val phe val arg asp cys phe

          *           *           *           *           *           *           *
GAG CAT CAC TGG GAT ACC TTA GAT TCT ACT CTC GAC AAA GTA GCC AAT ACC TCG GTT CCT TTA ATC GCC TTT ACC
glu his his trp asp thr leu asp ser thr leu asp lys val ala asn thr ser val pro leu ile ala phe thr

    *           *           400*                      *           *           *           *
GCT AAC AAC GAT GAT TGG GTT AAG CAA GAA GAA GTC TAT GAC ATG TTA GCG CAT ATC CGC ACT GGG CAT TGC AAG
ala asn asn asp asp trp val lys gln glu glu val tyr asp met leu ala his ile arg thr gly his cys lys

          *           *           *           *           500*          *           *
CTC TAC TCC TTG CTT GGT AGC TCT CAT GAC TTG GGC GAA AAC TTG GTC GTG TTA CGT AAT TTT TAC CAA TCC GTC
leu tyr ser leu leu gly ser ser his asp leu gly glu asn leu val val leu arg asn phe tyr gln ser val

    *           *           *           *           *           *           *           600*
ACC AAA GCC GCC ATC GCA ATG GAT GGA GGC AGC TTA GAA ATC GAC GTC GAC TTT ATC GAG CCT GAT TTT GAA CAA
thr lys ala ala ile ala met asp gly gly ser leu glu ile asp val asp phe ile glu pro asp phe glu gln

          *           *           *           *           *           *           *
CTC ACC ATC GCG ACT GTG AAT GAA CGT CGC TTG AAA GCG GAA ATT GAA AGC CGT ACG CCA GAA ATG GCT TAG GTC
leu thr ile ala thr val asn glu arg arg leu lys ala glu ile glu ser arg thr pro glu met ala end
```

# FIG. 7A

```
     *           *       700*                *            *            *              *             *            *
TTATCGTAATACCAACAAATAAGGAAATGTT ATG AAA TTT GGA AAC TTC CTT CTC ACT TAT CAG CCA CCT GAG CTA TCT CAG
                                met lys phe gly asn phe leu leu thr tyr gln pro pro glu leu ser gln
                                |────────────────|        |─────────────────────────────|     glu
                                      SAP 1                          SAP 2

  *           *           *            *          800*           *            *            *
ACC GAA GTG ATG AAG CGA TTG GTT AAT CTG GGC AAA GCG TCT GAA GGT TGT GGC TTC GAC ACC GTT TGG TTG CTA
thr glu val met lys arg leu val asn leu gly lys ala ser glu gly cys gly phe asp thr val trp leu leu
                                         |────────────────|
                                              SAP 3

         *           *            *            *             *             *        900*
GAG CAC CAC TTC ACT GAA TTT GGG TTG TTA GGG AAT CCT TAT GTT GCT GCC GCA CAC CTA TTA GGT GCG ACA GAA
glu his his phe thr glu phe gly leu leu gly asn pro tyr val ala ala ala his leu leu gly ala thr glu
|─────────────────────||─────────────────────|        |────────────────────────────────────────────|
         SAP 4              SAP 5                                      SAP 6

  *           *           *            *              *             *            *            *
ACG CTC AAC GTT GGC ACT GCA GCT ATC GTA TTG CCG ACT GCC CAT CCG GTT CGA CAA GCA GAA GAC GTA AAC CTA
thr leu asn val gly thr ala ala ile val leu pro thr ala his pro val arg gln ala glu asp val asn leu
|─────────────────────||─────────────────────────(───────────────)────────────────────────||──────
      SAP 7                              SAP 8 & 9

       *        1000*              *              *            *            *             *
CTG GAT CAA ATG TCA AAA GGA CGA TTC CGT TTT GGT ATT TGT CGC GGT TTG TAC GAT AAA GAT TTT CGT GTC TTT
leu asp gln met ser lys gly arg phe arg phe gly ile cys arg gly leu tyr asp lys asp phe arg val phe
────(──────────────────────)─────────────────||──────────────────────────||────────────────────
      SAP 10 & 11                                        SAP 12                  SAP 13 & 14

  *           *            *              *          1100*           *            *            *
GGT ACA GAC ATG GAT AAC AGC CGA GCC TTA ATG GAC TGT TGG TAT GAC TTG ATG AAA GAA GGC TTC AAT GAA GGC
gly thr asp met asp asn ser arg ala leu met asp cys trp tyr asp leu met lys glu gly phe asn glu gly
───|                                     |────────────────────||────────────||─────────────────||──
                                                   SAP 15           SAP 16
```

# FIG. 7B

```
        *                  *              *           *              *              *              1200*
TAT ATC GCG GCG GAT AAC GAA CAT ATT AAG TTC CCG AAA ATC CAA CTG AAT CCA TCG GCT TAC ACA CAA GGT GGT
tyr ile ala ala asp asn glu his ile lys phe pro lys ile gln leu asn pro ser ala tyr thr gln gly gly
_____|                                                       |_____
        SAP 17

        *              *          *              *              *              *              *              *
GCT CCT GTT TAT GTC GTC GCG GAG TCA GCA TCA ACG ACA GAA TGG GCT GCA GAG CGT GGC CTA CCA ATG ATT CTA
ala pro val tyr val val ala glu ser ala ser thr thr glu trp ala ala glu arg gly leu pro met ile leu
    ala                    pro   |                              |_____|
_____|
        SAP 18                                                      SAP 19

        *              1300*          *              *              *              *              *
AGC TGG ATC ATC AAC ACT CAC GAG AAG AAA GCG CAG CTT GAT CTT TAC AAC GAA GTC GCG ACT GAA CAT GGC TAC
ser trp ile ile asn thr his glu lys lys ala gln leu asp leu tyr asn glu val ala thr glu his gly tyr
                                    |_____|  |_____|  |_____|
                                            SAP 20                  SAP 21          SAP 22

        *              *              *              *              1400*          *              *
GAT GTG ACT AAG ATT GAC CAC TGT TTG TCT TAC ATC ACC TCC GTC GAT CAT GAC TCA AAT AGA GCC AAA GAT ATT
asp val thr lys ile asp his cys leu ser tyr ile thr ser val asp his asp ser asn arg ala lys asp ile
                                                                             |_____
                                                                                    T1

        *              *              *              *              *              *              1500*
TGC CGC AAC TTC TTG GGC CAT TGG TAC GAC TCA TAC GTG AAT GCC ACC AAG ATT TTT GAC GAC TCT GAC CAA ACA
cys arg asn phe leu gly his trp tyr asp ser tyr val asn ala thr lys ile phe asp asp ser asp gln thr
_____|             |_____|  |_____|
                            SAP 23                  SAP 24

        *              *              *              *              *              *              *
AAA GGT TAC GAC TTC AAT AAA GGT CAA TGG CGT GAT TTT GTG TTG AAA GGC CAC AAA GAC ACC AAT CGC CGA ATT
lys gly tyr asp phe asn lys gly gln trp arg asp phe val leu lys gly his lys asp thr asn arg arg ile
                                        |_____|  |_____
                                                SAP 25              SAP 26 & T2
```

<div align="center">

# FIG. 7C

</div>

```
         *         1600*                    *              *              *              *              *              *
GAT TAC AGC TAC GAA ATC AAC CCA GTA GGG ACG CCT GAA GAG TGT ATC GCG ATT ATC CAG CAA GAT ATT GAT GCG
asp tyr ser tyr glu ile asn pro val gly thr pro glu glu cys ile ala ile ile gln gln asp ile asp ala
─────────(─────────) |───────────────────────────────────────────────────────────────────────────────|
                                    SAP 27


  *              *              *              *           1700*              *              *              *
ACG GGT ATT GAC AAT ATT TGT TGT GGT TTT GAA GCA AAC GGT TCT GAA GAA GAA ATT ATC GCA TCT ATG AAG CTA
thr gly ile asp asn ile cys cys gly phe glu ala asn gly ser glu glu glu ile ile ala ser met lys leu
                                             |─────────────────────────────────||──────────────────────
                                                          SAP 28                     SAP 29, 30 & 31


         *              *              *           *           *        *        1800*              *
TTC CAG TCT GAT GTG ATG CCA TAT CTC AAA GAA AAA CAG TAATTAATATTTTCTAAAAGGAAAGAGACATG AAA TTT GGA
phe gln ser asp val met pro tyr leu lys glu lys gln end                            met lys phe gly
───────────────||──────────────────────────────────||─────────|
                   SAP 32                               SAP 33


         *              *
TTA TTC TTC CTC AAT TTT ATG AAT TC
leu phe phe leu asn phe met asn
```

# FIG.7D